(19) Europäisches Patentamt / European Patent Office / Office européen des brevets

(11) **EP 4 635 513 A1**

(12) **EUROPEAN PATENT APPLICATION**
published in accordance with Art. 153(4) EPC

(43) Date of publication:
**22.10.2025 Bulletin 2025/43**

(21) Application number: **25737533.7**

(22) Date of filing: **28.04.2025**

(51) International Patent Classification (IPC):
***A61K 41/00*** *(2020.01)* ***A61K 49/00*** *(2006.01)*
***A61K 49/22*** *(2006.01)* ***A61P 35/00*** *(2006.01)*
***C07D 498/04*** *(2006.01)* ***C07D 498/06*** *(2006.01)*
***G01N 21/64*** *(2006.01)*

(86) International application number:
**PCT/CN2025/091672**

(87) International publication number:
**WO 2025/176229 (28.08.2025 Gazette 2025/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **30.04.2024 CN 202410541675**

(71) Applicant: **Shenzhen University**
**Shenzhen, Guandong 518061 (CN)**

(72) Inventors:
- **LIN, Jing**
  **Shenzhen, Guangdong 518061 (CN)**
- **LIU, Yurong**
  **Shenzhen, Guangdong 518061 (CN)**
- **HUANG, Peng**
  **Shenzhen, Guangdong 518061 (CN)**

(74) Representative: **Isern Patentes y Marcas S.L.**
**Avda. Diagonal, 463 Bis, 2°**
**08036 Barcelona (ES)**

# (54) NEAR-INFRARED PHOTOSENSITIZER THAT CAN BE ACTIVATED IN SLIGHTLY ACIDIC TUMOR ENVIRONMENT AND PREPARATION METHOD THEREFOR AND USE THEREOF

(57) The present disclosure relates to the field of biomedicine synthesis technology, in particular to an acidic tumor microenvironment activatable near-infrared photosensitizer, as well as preparation methods therefore and applications thereof. The acidic tumor microenvironment activatable near-infrared photosensitizer has a chemical structure represented by any one of formulas (I) to (VI). The acidic tumor microenvironment activatable near-infrared photosensitizer employs cyanine or hemi-cyanine derivatives as the fundamental molecular scaffold exhibiting superior biocompatibility and high near-infrared absorption capacity with an ether-oxygen bond incorporated at the ortho-position of the indole nitrogen atom as an acid-responsive group. The reactive oxygen species generation capability of the photosensitizer molecule is modulated through substitution of different halogen atoms at R1 position, and while R2 group is hydroxyl, amino or biomarker-responsive groups. The photosensitizer of the present disclosure is specifically activated in tumors, exhibits intense near-infrared absorption and fluorescence emission characteristics with effective photodynamic therapeutic efficacy to enable precise tumor-targeted treatment with minimized systemic toxicity, thereby providing novel strategic potential for precision oncology therapeutics.

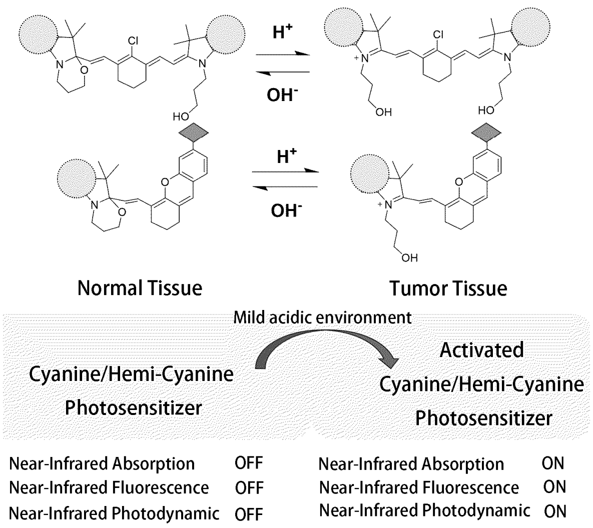


FIG. 1

EP 4 635 513 A1

## Description

### FIELD

The present disclosure relates to the field of biomedicine synthesis technology, in particular to an acidic tumor microenvironment activatable near-infrared photosensitizer, as well as preparation methods therefore and applications thereof.

### BACKGROUND

Cancer exhibits high incidence and mortality rates, posing severe threats to human health. Photodynamic therapy (PDT) represents an emerging oncological treatment modality that utilizes photosensitizers to transfer laser energy to tumor-dissolved oxygen, generating cytotoxic singlet oxygen capable of killing tumor cells. This phototherapy approach demonstrates the following advantages: (1) Minimal tissue trauma: The treatment approach achieves non-invasiveness through light irradiation, and can guide laser to a deep part of human body facilitated through optical fibers, endoscopes, and other interventional techniques, thereby obviating surgical trauma and pain from thoracotomy, laparotomy, etc; (2) Superior spatiotemporal selectivity with negligible systemic toxicity: Photosensitizing agents entering in tissues only induce photodynamic reaction to kill targeted cells upon reaching a certain concentration and receiving sufficient light irradiation, and the part of the photosensitizing agents without light irradiation does not produce this killing reaction, thereby minimizing damage to other organs and tissues and not affecting hematopoietic function, with this selective localized irradiation treatment approach demonstrating significantly reduced systemic adverse effects unattainable by many other treatment approach; (3) Broad applicability: Unlike radiotherapy and chemotherapy exhibit significant variability in treatment response across different tumor cell types resulting in limited clinical utility, PDT demonstrates consistent effectiveness against neoplastic lesions of diverse cellular origins, thereby providing broad applicability; (4) Repeatable treatment: Tumor cells develop no drug resistance to the photosensitizing agents, and patients exhibit no cumulative toxicity upon multiple treatments, thereby enabling repeatable treatment.

The photosensitizers currently used in clinical practice are porphyrin derivatives, which exhibit the following limitations: (1) The non-selective systemic distribution of photosensitizer results in excessive accumulation within normal tissues, inducing clinical adverse reactions such as cutaneous phototoxicity that impair life quality of patient. As exemplified by Photofrin®, currently one of the most widely used clinical photosensitizer agents, with hematoporphyrin derivatives as primary components, demonstrates deficient tumor selectivity, and diffuses extensively throughout cutaneous tissues after administration, causing severe photodermatitis upon solar exposure. The FDA (Food and Drug Administration) requires sunlight avoidance for skin and eyes for up to one month post-administration, significantly affecting lifestyle of patient. (2) The limited laser penetration depth of the photosensitizers results in suboptimal therapeutic efficacy against deep-seated tumors; (3) The generally low reactive oxygen species (ROS) generation efficiency of the photosensitizers, compounded by the inherently hypoxic tumor microenvironment, and the progressive oxygen depletion during treatment, diminishes photodynamic therapy efficacy. Consequently, the administration of a high dose of photosensitizer dosages doses is necessitated to satisfy therapeutic requirements, increasing long-term safety risks of medication. Consequently, overcoming these limitations and developing novel photosensitizer molecular architectures holds significant clinical importance for advancing photodynamic therapy applications.

Therefore, the prior art still requires improvement and development.

### SUMMARY

In view of the deficiencies in the prior art described above, an object of the present disclosure is to provide an acidic tumor microenvironment activatable near-infrared photosensitizer, as well as preparation method and application thereof, to solve the problems of the photosensitizers in the prior art, such as high cutaneous phototoxicity, limited treatment depth, and low reactive oxygen species generation efficiency.

The technical solutions of the present disclosure are as follows:

In a first aspect of the present disclosure, a series of the near-infrared photosensitizers activatable in acidic tumor microenvironment is provided, having a chemical structural formula as shown in any one of formulas (I) to (VI):

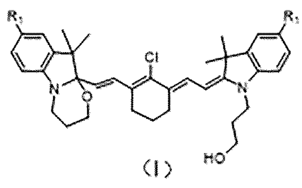
(I)
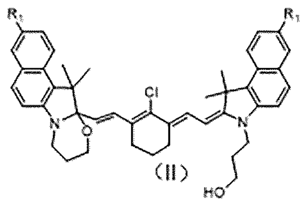
(II)
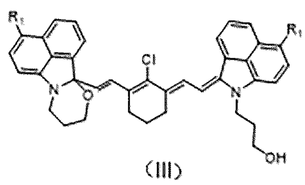
(III)

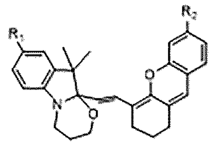
(IV)
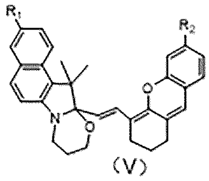
(V)
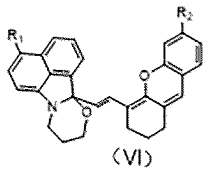
(VI)

;

$R_1$ is a hydrogen, fluorine, chlorine, bromine, or iodine atom; $R_2$ is -OH, $-NH_2$,

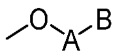
,
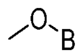
, or
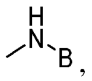
,

A is a self-eliminating group, B is a biomarker-responsive group.

The photosensitizer provided by the present disclosure is based on a cyanine/hemi-cyanine parent structure, with an ether-oxygen bond incorporated at ortho-position of indole nitrogen atom to serve as an acid-responsive group. The ability to generate reactive oxygen species by photosensitizer molecules is modulated through substitution of different halogen atoms (fluorine, chlorine, bromine, and iodine) at the R1 position. For the hemicyanine scaffold, in addition to the acid-responsive group, the hemi-cyanine-based photosensitizer molecule is further modified at one terminus with an $R_2$ group selected from hydroxyl, amino, or biomarker-responsive groups. The cyanine molecular scaffold of Formulas (I) to (III) exhibits high molar extinction coefficients and near-infrared photosensitive properties, with the inherent positive charge of the indole structure enabling mitochondrial targeting capability. The hemi-cyanine molecular scaffold of Formulas (IV) - (VI), as structural variant of the cyanine molecule, exhibits photophysical and photochemical properties closely resembling the corresponding properties of cyanine molecule, while demonstrating enhanced photostability and structural modifiability. The acid-responsive hydroxyl group forms a closed-loop structure via the ether-oxygen bond at the ortho-position of the indole nitrogen atom, rendering the photosensitizer in an inactivated state, and exhibiting no near-infrared absorption, fluorescence emission, or near-infrared photodynamic therapeutic efficacy. However, upon activation in the acidic tumor microenvironment, cleavage of the acid-responsive ether-oxygen bond forms an open-ring configuration, restoring the native cyanine/hemi-cyanine architecture, thereby enabling intense near-infrared absorption, fluorescence emission, and near-infrared photodynamic therapeutic efficacy. Halogen atom substitutions at $R_1$ position of the photosensitizer differentially influence reactive oxygen species (ROS) generation yield with different halogen atoms demonstrating distinct atomic effects during molecular photosensitization process. Incorporation of a biomarker-responsive group $R_2$ into the hemi-cyanine scaffold, in combination with the acid-responsive group, yields a dual-responsive photosensitizer with enhanced tumor selectivity.

It is to be noted that

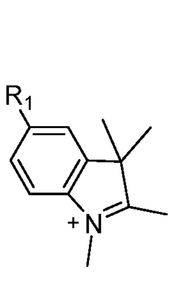
,
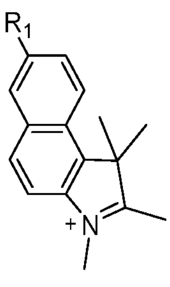
and
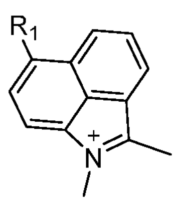

in chemical structure formulas of the photosensitizer are all indole structures that can be interchangeable in the present disclosure. However, progressively increasing conjugation degree in the molecular scaffold leads to corresponding bathochromic shifts in both absorption and emission wavelengths.

Optionally, the self-eliminating group A is

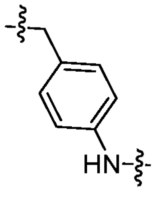, 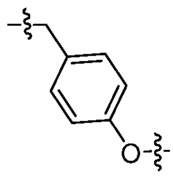,

or

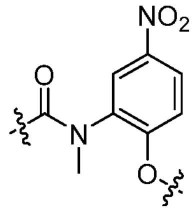.

Optionally, the biomarker-responsive group B is selected from:

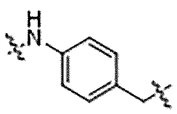, 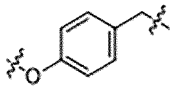, 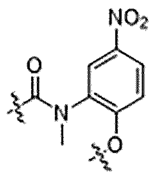, 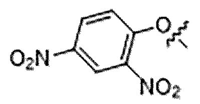,

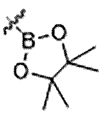, 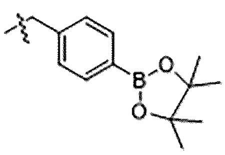, 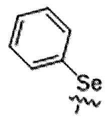, 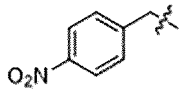, 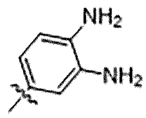,

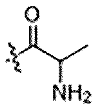, 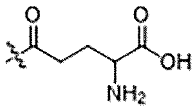, 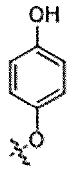, 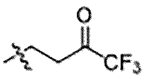, 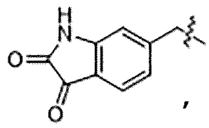,

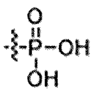, 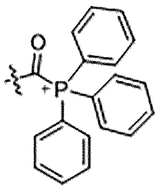, 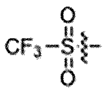, 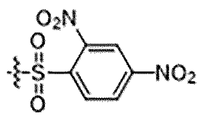, 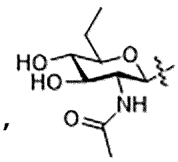,

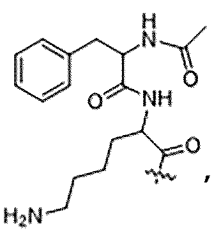, 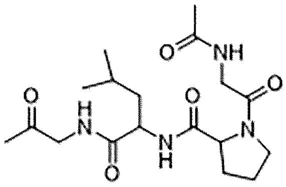 or 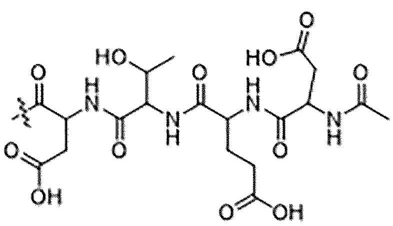.

Optionally, the photosensitizers represented by formulas (I) to (VI), upon activation in a mild acidic environment, exhibit corresponding structures in sequence represented by formulas (I') to (VI'):

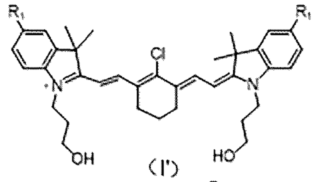
(I')
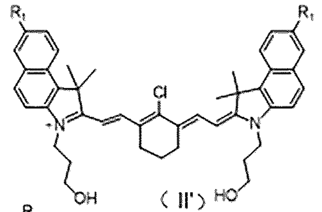
(II')
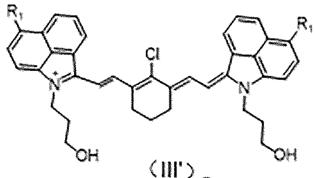
(III')
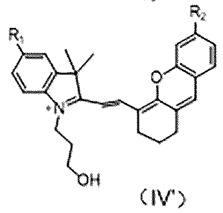
(IV')
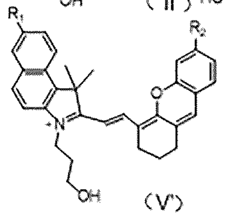
(V')
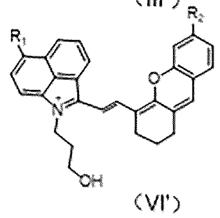
(VI')

.

Optionally, the acidic tumor microenvironment activatable near-infrared photosensitizer has structures represented by following formulas:

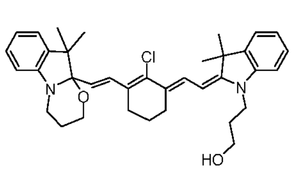
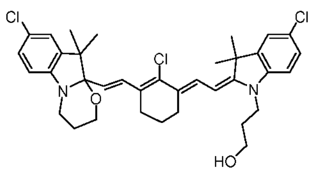
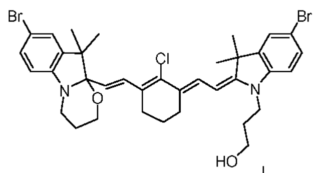
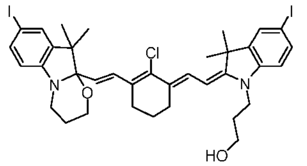
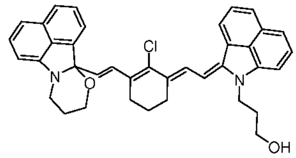
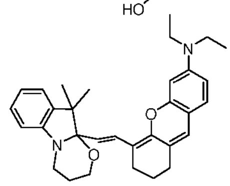
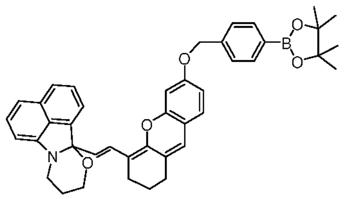
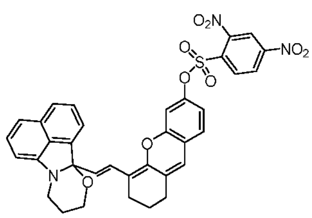

The applications of the acidic tumor microenvironment activatable near-infrared photosensitizer in the present disclosure, or pharmaceutically acceptable enantiomers, diastereomers, and tautomers thereof, in the preparation of photosensitizing agents are also within protection scope of the present disclosure.

In a second aspect of the present disclosure, a method for preparing the acidic tumor microenvironment activatable near-infrared photosensitizer is provided, including steps of:

S1, performing a condensation reaction between compound 1 represented by formula 1 and compound 4 represented by formula 4 to obtain compound 6 represented by formula 6;

or performing the condensation reaction between compound 2 represented by formula 2 and the compound 4 represented by the formula 4 obtain compound 7 represented by formula 7;

or performing the condensation reaction between compound 3 represented by formula 3 and the compound 4 represented by the formula 4 to obtain compound 8 represented by formula 8;

or performing the condensation reaction between the compound 1 represented by the formula 1 and compound 5 represented by formula 5 to obtain compound 9 represented by formula 9;

or performing the condensation reaction between the compound 2 represented by the formula 2 and the compound 5 represented by the formula 5 to obtain compound 10 represented by formula 10;

or performing the condensation reaction between the compound 3 represented by the formula 3 and the compound 5 represented by the formula 5 to obtain compound 11 represented by formula 11; and,

S2, subjecting the compound 6 to sequential ester hydrolysis reaction and ring-closure reaction under alkaline conditions to obtain the acidic tumor microenvironment activatable near-infrared photosensitizer represented by formula (I);

or subjecting the compound 7 to sequential the ester hydrolysis reaction and the ring-closure reaction under the alkaline conditions to obtain the acidic tumor microenvironment activatable near-infrared photosensitizer represented by formula (II);

or subjecting the compound 8 to the ring-closure reaction under the alkaline conditions to obtain the acidic tumor microenvironment activatable near-infrared photosensitizer represented by formula (III);

or subjecting the compound 9 to sequential the ester hydrolysis reaction and the ring-closure reaction under the alkaline conditions to obtain the acidic tumor microenvironment activatable near-infrared photosensitizer represented by formula (IV);

or subjecting the compound 10 to sequential the ester hydrolysis reaction and the ring-closure reaction under the alkaline conditions to obtain the acidic tumor microenvironment activatable near-infrared photosensitizer represented by formula (V);

or subjecting the compound 11 to the ring-closure reaction under the alkaline conditions to obtain the acidic tumor microenvironment activatable near-infrared photosensitizer represented by formula (VI);

chemical structural formulas of the compound 1 to the compound 11 are represented by the formula 1 to the formula 11 as shown below:

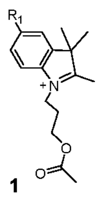
1

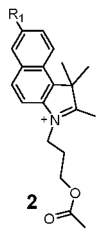
2

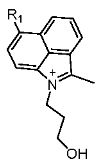
3

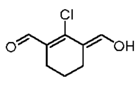
4

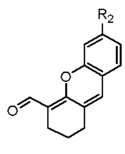
5

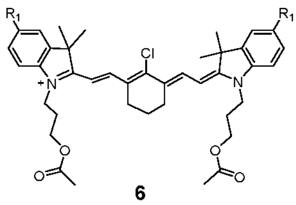
6

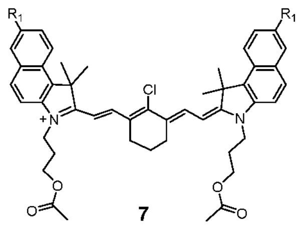
7

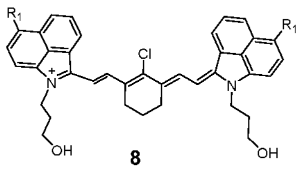
8

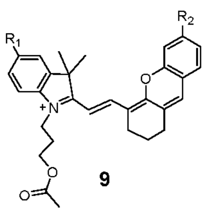
9

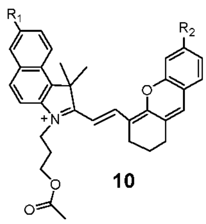
10

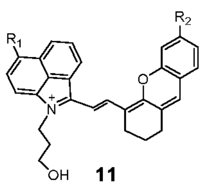
11

.

Optionally, a temperature of the condensation reaction in the step S1 is 50 to 100 °C and a duration of the condensation reaction is 2 to 12 hours;

Optionally, the solvent employed in the condensation reaction of step S1 is one or more of acetic anhydride, anhydrous methanol, anhydrous ethanol, and anhydrous N,N-dimethylformamide, either individually or in combination; and an activator is optionally one or more of sodium acetate, potassium acetate, potassium carbonate, and cesium carbonate.

Optionally, the alkaline condition required for the ring-closure reaction in the step S2 is one or more of potassium carbonate, cesium carbonate, potassium hydroxide, sodium hydroxide, sodium hydride, aqueous ammonia, and triethylamine;

Optionally, a solvent employed in the ring-closure reaction in the step S2 is one or more of dichloromethane, methanol, ethanol, chloroform, and N,N-dimethylformamide.

Optionally, a temperatur of the ring-closure reaction is 0 to 50 °C, and a duration of the ring-closure reaction is 2 to 12 hours.

Optionally, the compound 1 in the step S1 is prepared by a substitution reaction between the compound 12 and the compound 13. A temperature of the substitution reaction is 50 °C to 150 °C, and a duration of the substitution reaction is 24 to 72 hours; and/or, a solvent employed in the substitution reaction is one or more of acetonitrile, N,N-dimethylformamide, dichloromethane, chloroform, toluene, and o-dichlorobenzene;

the chemical structural formulas of the compound 12 and the compound 13 are as shown below:

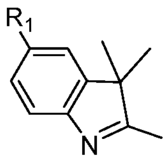

**12**

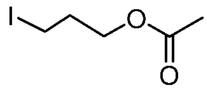

**13**

.

Optionally, the compound 3 in the step S1 is prepared through following steps:

subjecting compound 14 to a sulfuration reaction to obtain compound 15;

subjecting the compound 15 to an iodination reaction to obtain compound 16;

reacting the compound 16 with compound 17 via the substitution reaction to obtain compound 18;

reacting the compound 18 with the compound 13 via the substitution reaction to obtain compound 19;

subjecting the compound 19 to a hydrolysis reaction to obtain the compound 3;

the chemical structural formulas of the compounds 13 to 19 are as shown below:

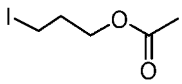

**13**

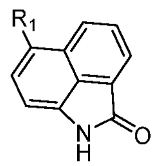

**14**

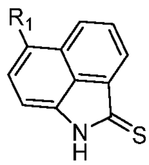

**15**

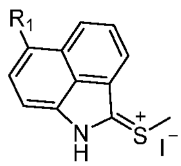

**16**

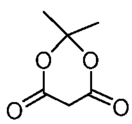

**17**

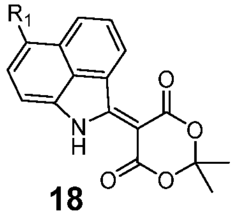

**18**

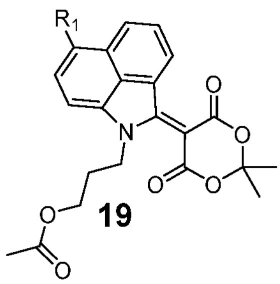

**19**

.

Optionally, the reaction involved in the preparation step of Compound 3 is conducted at a temperature ranging from 50 °C to 150 °C for a duration of 24 to 72 hours, and a solvent employed in the reaction is one or more of acetonitrile, N,N-dimethylformamide, dichloromethane, chloroform, toluene, and o-dichlorobenzene.

In a third aspect of the present disclosure, an application of the acidic tumor microenvironment activatable near-infrared photosensitizer for preparing a tumor diagnostic agent and/or a tumor therapeutic agent, is provided.

Optionally, the tumor diagnostic agent includes a fluorescence imaging agent and/or a photoacoustic imaging agent.

Optionally, the tumor therapeutic agent includes a photodynamic therapy agent or a combination therapy agent for photodynamic therapy with other treatment regimens.

Optionally, the pharmaceutical formulation is in a dosage form selected from capsule, tablet, oral preparation, injection, suppositorie, spray, and ointment.

Near-infrared dye molecules activatable in acidic tumor microenvironment provided by the present disclosure can serve as a photosensitizer for photodynamic therapy, and be formulated into pharmaceutical preparations adapted for various administration routes including intravenous injection, microneedle patches, intraperitoneal injection, or spray application. When the acidic tumor microenvironment activatable near-infrared photosensitizer is intravenously administered, the acid-responsive group of the photosensitizer molecules maintain a closed-ring configuration under a physiological pH 7.4 microenvironment of normal tissues, thereby rendering the photosensitizer molecules in an inactivated state that precludes detectable near-infrared absorption or emission in normal tissues, and further preventing reactive oxygen species generation upon near-infrared irradiation, thus avoiding damage to normal tissues. When the photosensitizer molecules are in the acidic tumor microenvironment ($pH < 7$), the acid-responsive groups adopt an open-ring configuration, activating the photosensitizer molecules represented by Formulas (I) to (III) to exhibit intense near-infrared absorption and emission suitable for fluorescence or photoacoustic imaging, and further generating reactive oxygen species upon near-infrared irradiation to effect tumor tissue ablation. The photosensitizer molecules represented by Formulas (IV) to (VI), upon further activation by tumor-specific biomarkers abundantly expressed in tumors, exhibit near-infrared absorption and emission enabling fluorescence or photoacoustic imaging-guided photodynamic therapy, thereby achieving precision visualized photodynamic therapy with enhanced therapeutic efficacy, and providing a novel approach for the development of photodynamic therapy.

## BRIEF DESCRIPTION OF THE DRAWINGS

FIG. 1 is a schematic diagram illustrating an activation mechanism of a acidic tumor microenvironment activatable near-infrared photosensitizer according to the present disclosure.

FIG. 2 shows synthetic routes for activatable near-infrared photosensitizers LET-H, LET-Cl, LET-Br, and LET-I (collectively designated as LET-R) according to Embodiments 1-4 of the present disclosure.

FIG. 3 shows a synthetic route of activatable near-infrared photosensitizer LET-BCy according to Embodiment 5 of the present disclosure.

FIG. 4 shows a synthetic route of activatable near-infrared photosensitizer LET-Hcy-N according to Embodiment 6 of the present disclosure.

FIG. 5 shows synthetic routes of activatable near-infrared photosensitizers LET-BHcy-ROS and LET-BHcy-GSH according to Embodiments 7-8 of the present disclosure.

FIG. 6 is pH-response absorption spectra of activatable near-infrared photosensitizers according to Embodiments 1-8 of the present disclosure.

FIG. 7 is fluorescence emission spectra, (a) and (b) show pH-response fluorescence spectra of LET-H at excitation wavelengths of 420 nm and 760 nm; (c) and (d) show pH-response fluorescence spectra of LET-I at excitation wavelengths of 420 nm and 760 nm; (e) shows the pH-response fluorescence spectrum of LET-Hcy-N at an excitation wavelength of 660 nm.

FIG. 8 presents analytical data of photosensitizers LET-R' (R = H, Cl, Br, I), representing activated molecules of LET-R, (a) shows comparative degradation rates of DPBF by LET-R and LET-R' under 808 nm laser irradiation (power: 0.2 W $cm^{-2}$), (b) shows comparative singlet oxygen quantum yields of LET-R', and (c) shows electron paramagnetic resonance spectra detecting singlet oxygen generation by LET-H' and LET-I' in irradiated and non-irradiated conditions.

FIG. 9 shows cell-killing efficacy test results of activatable near-infrared photosensitizers LET-H and LET-I under different irradiation durations.

In FIG. 10, (a) shows fluorescence imaging of folate-modified nanoengineered LET-I (designated LET-I-FA) in murine tumor and leg tissues, (b) shows corresponding fluorescence intensity changes over time, (c) shows photoacoustic imaging of LET-I-FA in murine tumor and leg tissues, and (d) shows corresponding photoacoustic signal intensity changes over time.

In FIG. 11, (a) shows therapeutic effect evaluation of photosensitizer LET-I-FA in 4T1 tumor-bearing mice; (b) shows statistical tumor weight data of mice post-treatment; (c) shows body weight changes of each group during treatment, and LET-H-FA indicates folate-modified nanoengineered photosensitizer LET-H.

In FIG. 12, (a) shows histological sections of major organs from each group post-treatment, (b) shows hematological biochemistry evaluation results for each group.

## DETAILED DESCRIPTION OF EMBODIMENTS

The present disclosure provides an acidic tumor microenvironment activatable near-infrared photosensitizer, as well as preparation methods and applications thereof. To clarify and define the objectives, technical solutions, and effects of the present disclosure, the following provides a detailed description of the present disclosure. It shall be understood that the specific embodiments described herein are provided solely to illustrate the present disclosure and should not be construed as limiting the scope of the present disclosure.

Unless otherwise defined, all technical and scientific terms used herein have the same meanings as commonly understood by those skilled in the art to which the present disclosure pertains. The terminology used in the description of the present disclosure herein is for the purpose of describing embodiments only and is not intended to limit the present disclosure.

The present disclosure is described in detail through following specific embodiments.

Embodiment 1

A synthetic route for preparing a near-infrared photosensitizer LET-H activatable in acidic tumor microenvironment in the embodiment is shown in FIG. 2, with synthetic steps as follows:

Under nitrogen atmosphere protection, phosphorus oxychloride (8.7 mL, 58 mmol) was slowly added to a solution of 10 mL dichloromethane and 10 mL N,N-dimethylformamide. A reaction mixture was stirred in an ice bath for 30 minutes, followed by addition of Compound A1 (2.5 g, 25 mmol). After stirring at 80 °C for 6 hours and cooling to room temperature, then the reaction mixture was treated with ice water and stirred overnight. The reqaction mixture was filtered, and a precipitate was sequentially washed with a small amount of ice water followed by ice-cold ethanol, then dried in an oven to afford Compound B1 as a yellow solid (4.1 g, 96% yield). $^1$H NMR (400 MHz, DMSO-$d_6$) $\delta$ 10.85 (s, 1H), 2.36 (m, 4H), 1.59 (m, 2H).

Compound D1 (1.07 g, 4.71 mmol) was added dropwise to a solution of Compound C1 (500 mg, 3.14 mmol) in acetonitrile, and refluxed for 1 day. After cooling, the reaction mixture was poured into a mixture of 22.5 mL dichloromethane and 2.5 mL methanol. Then 10 mL cold diethyl ether was added to the reaction mixture, and a crystalline precipitate was collected and washed with 3 $\times$ 3 mL diethyl ether, followed by vacuum drying to afford Compound E1 as a light brown solid (1.055 g, 87% yield). $^1$H NMR (400 MHz, $CDCl_3$) $\delta$ 7.73 (m, 1H), 7.59 (m, 3H), 4.89 (t, *J* = 8 Hz, 2H),4.23 (t, *J* = 4 Hz, 2H), 3.17 (s, 3H), 2.38 (m, 2H), 2.00 (s, 3H), 1.67 (s, 6H). HRMS (ESI) calcd for $C_{16}H_{22}NO_2^+$ 260.1645, found 260.1636.

Under nitrogen atmosphere protection, the Compound B1 (107 mg, 0.65 mmol) and Compound E1 (500 mg, 1.3 mmol) and sodium acetate (106 mg, 1.3 mmol) were added to 5 mL acetic anhydride. The reaction mixture was heated to 70 °C and reacted for 2 hours. The reaction mixture was then poured into cold diethyl ether, and a precipitated solid was collected to afford Compound F1 as a metallic green-lustrous solid (491 mg, 96% yield). $^1$H NMR (500 MHz, DMSO-$d_6$) $\delta$ 8.29 (d, *J* = 10 Hz, 2H), 7.66 (d, *J* = 10 Hz, 2H), 7.45 (m, 4H), 7.30 (m, 2H), 6.36 (d, *J* = 15 Hz, 2H), 4.30 (t, *J* = 7 Hz, 4H), 4.09 (t, *J* = 6 Hz, 4H), 2.72 (t, *J* = 5 Hz, 4H), 2.08 (m, 4H), 1.94 (s, 6H), 1.86 (m, 2H), 1.69 (s, 12H). HRMS (ESI) calcd for $C_{40}H_{48}ClN_2O_4^+$ 655.3297, found 655.3275.

Under nitrogen atmosphere protection, the Compound F1 (464 mg, 0.59 mmol) was dissolved in 30 mL methanol, followed by addition of solid potassium carbonate (164 mg, 1.19 mmol). The reaction mixture was stirred at room temperature (25 $\pm$ 2°C) for 3 hours, through cyclization of intermediate Compound G1, a final product was synthesized. A solvent was removed by rotary evaporation, and 10 mL of dichloromethane was added. The reaction mixture was washed twice with 10 mL saturated aqueous sodium bicarbonate solution, and dried over anhydrous sodium

sulfate, and the solvent was then removed by rotary evaporation. A crude product was purified by alumina column chromatography (eluent: dichloromethane) to afford the near-infrared photosensitizer LET-H activatable in acidic tumor microenvironment as a pale yellow solid (208 mg, 62% yield). $^1$H NMR (400 MHz, DMSO-$d_6$) δ 8.27 (d, $J$ = 8 Hz, 2H), 7.64 (d, $J$ = 4 Hz, 2H), 7.45 (m, 4H), 7.29 (td, $J_1$ = 4.6 Hz, $J_2$ = 1.2 Hz, 2H), 6.44 (d, $J$ = 8 Hz, 2H), 4.87 (bro, 2H), 4.26 (t, $J$ = 4 Hz, 4H), 3.50 (bro, 4H), 2.70 (t, $J$ = 4 Hz, 4H), 1.90 (m, 4H), 1.68 (m, 14H). $^{13}$C NMR (100 MHz, $CDCl_3$) δ 172.6, 144.7, 142.1, 139.2, 128.9, 127.2, 125.3, 122.1, 119.2, 110.8, 108.7, 68.2, 65.7, 62.3, 58.0, 41.2, 40.0, 29.9, 29.6, 28.1, 26.2, 22.0, 20.6, 18.3. HRMS (ESI) calcd for $C_{36}H_{43}ClN_2O_2^+$ 570.3013, found $[M+H]^+$ 571.3078.

Embodiment 2

A synthetic route for preparing a near-infrared photosensitizer LET-Cl activatable in acidic tumor microenvironment in the embodiment is shown in FIG. 2, with synthetic steps as follows:
Compound E2 was synthesized as a brown solid in 78% yield from Compound C2 and the Compound D1 using the same synthetic procedure as described in the Embodiment 1.$^1$H NMR (500 MHz, $CDCl_3$) δ 7.88 (d, $J$ = 5 Hz, 1H), 7.52 (s, 1H), 7.51 (d, $J$ = 2 Hz, 1H), 4.81 (t, $J$ = 5 Hz, 2H), 4.20 (t, $J$ = 5 Hz, 2H), 3.11 (s, 3H), 2.35 (m, 2H), 1.97 (s, 3H), 1.65 (s, 6H).
Compound F2 was obtained as a green solid in 92% yield from the Compound E2 using the same synthetic procedure as described in the Embodiment 1. $^1$H NMR (500 MHz, $CDCl_3$) δ 8.33 (d, $J$ = 10 Hz, 2H), 7.37 (dd, $J_1$ = 7 Hz, $J_2$ = 2 Hz, 2H), 7.33 (d, $J$ = 2 Hz, 2H), 7.12 (d, $J$ = 7 Hz, 2H), 6.34 (d, $J$ = 10 Hz, 2H), 4.38 (t, $J$ = 6 Hz, 4H), 4.18 (t, $J$ = 5 Hz, 4H), 2.79 (t, $J$ = 5 Hz, 4H), 2.21 (m, 4H), 2.04 (s, 6H), 1.98 (m, 2H), 1.73 (s, 12H).
The Compound F2 was subjected to the same synthetic procedure as described in the Embodiment 1 through cyclization of intermediate Compound G2 to afford a yellow solid in 58% yield, which was the acidic tumor microenvironment activatable near-infrared photosensitizer, designated as LET-Cl. $^1$H NMR (500 MHz, $CD_3OD$) δ 8.45 (d, $J$ = 15 Hz, 2H), 7.59 (d, $J$ = 5 Hz, 2H), 7.44 (dd, $J_1$ = 10 Hz, $J_2$ = 5 Hz, 2H), 7.36 (d, $J$ = 10 Hz, 2H), 6.45 (d, $J$ = 15 Hz, 2H), 4.25 (t, $J$ = 10 Hz, 4H), 3.65 (t, $J$ = 5 Hz, 4H), 2.74 (t, $J$ = 5 Hz, 4H), 2.03 (m, 4H), 1.95 (m, 2H), 1.74 (s, 12H). $^{13}$C NMR (100 MHz, $CD_3OD$) δ 174.1, 145.6, 144.5, 142.5, 132.1, 129.9, 128.9, 124.1, 113.5, 59.3, 50.7, 42.5, 31.0, 28.2, 27.3, 22.1. HRMS (ESI) calcd for $C_{36}H_{41}Cl_3N_2O_2^+$ 640.2204, found $[M+H]^+$ 641.2263.

Embodiment 3

A synthetic route for preparing a near-infrared photosensitizer LET-Br activatable in acidic tumor microenvironment in the embodiment is shown in FIG. 2, with synthetic steps as follows:
Compound E3 was synthesized as a pale yellow solid in 82% yield from Compound C3 and the Compound D1 using the same synthetic procedure as described for preparation of the Compound E1 in the Embodiment 1. $^1$H NMR (500 MHz, $CDCl_3$) δ 7.80 (d, $J$ = 5 Hz, 1H), 7.71 (d, $J$ = 5 Hz, 1H), 7.68 (s, 1H), 4.85 (t, 2H), 4.22 (t, 2H), 3.12 (s, 3H), 2.36 (t, 2H), 2.00 (s, 3H), 1.67 (s, 6H).
Compound F3 was obtained as a green solid in 95% yield from the Compound E3 using the same synthetic procedure as described in the Embodiment 1. $^1$H NMR (500 MHz, $CDCl_3$) δ 8.34 (d, $J$ = 15 Hz, 2H), 7.52 (dd, $J_1$ = 7 Hz, $J_2$ = 1.5 Hz, 2H), 7.47 (d, $J$ = 1.5 Hz, 2H), 7.07 (d, $J$ = 5 Hz, 2H), 6.34 (d, $J$ = 10 Hz, 2H), 4.37 (t, $J$ = 5.5 Hz, 4H), 4.18 (t, $J$ = 4.5 Hz, 4H), 2.79 (t, $J$ = 5 Hz, 4H), 2.22 (m, 4H), 2.04 (s, 6H), 1.97 (m, 2H), 1.73 (s, 12H).
The Compound F3 was subjected to the same synthetic procedure as described in the Embodiment 1 through cyclization of intermediate Compound G3 to afford a yellow solid in 67% yield, which was the acidic tumor microenvironment activatable near-infrared photosensitizer, designated as LET-Br. $^1$H NMR (500 MHz, $CD_3OD$) δ 8.45 (d, $J$ = 15 Hz, 2H), 7.72 (d, $J$ = 2.5 Hz, 2H), 7.58 (dd, $J_1$ = 11 Hz, $J_2$ = 2.5 Hz, 2H), 7.31 (d, $J$ = 10.5 Hz, 2H), 6.46 (d, $J$ = 8 Hz, 2H), 4.25 (t, $J$ = 9 Hz, 4H), 3.65 (t, $J$ = 6.5 Hz, 4H), 2.74 (t, $J$ = 7 Hz, 4H), 2.02 (m, 4H), 1.95 (m, 2H), 1.74 (s, 12H). $^{13}$C NMR (100 MHz, $CD_3OD$) δ 172.6, 143.4, 141.5, 131.5, 127.5, 125.6, 118.0, 112.5, 101.6, 57.8, 49.3, 41.0, 29.6, 26.8, 25.9, 20.7. HRMS (ESI) calcd for $C_{36}H_{41}Br_2ClN_2O_2^+$ 728.1203, found $[M+H]^+$ 729.1257.

Embodiment 4

A synthetic route for preparing a near-infrared photosensitizer LET-I activatable in acidic tumor microenvironment in the embodiment is shown in FIG. 2, with synthetic steps as follows:
Compound E4 was synthesized as a brown solid in 60% yield from Compound C4 and the Compound D1 following a procedure analogous to that described in the Embodiment 1. $^1$H NMR (400 MHz, DMSO-$d_6$) δ 8.32 (s, 1H), 8.03 (d, $J$ = 8 Hz, 1H), 7.78 (d, $J$ = 8 Hz, 1H), 4.51 (t, $J$ = 8 Hz, 2H), 4.13 (t, $J$ = 4 Hz, 2H), 2.81 (s, 3H), 2.18 (m, 2H), 1.93 (s, 3H), 1.54 (s, 6H). HRMS (ESI) calcd for $C_{16}H_{22}INO_2^+$ 386.0611, found 386.0608.
Compound F4 was obtained as a green solid in 41% yield from the Compound E4 following the procedure analogous to that described in the Embodiment 1. $^1$H NMR (400 MHz, $CDCl_3$) δ 8.34 (d, $J$ = 11.2 Hz, 2H), 7.71 (dd, $J_1$ = 6.4 Hz, $J_2$ = 0.8 Hz, 2H), 7.65 (d, $J$ = 0.8 Hz, 2H), 6.97 (d, $J$ = 6.8 Hz, 2H), 6.36 (d, $J$ = 11.2 Hz, 2H), 4.37 (t, $J$ = 5.6 Hz, 4H), 4.18 (t,

$J$ = 4.8 Hz, 4H), 2.79 (t, $J$ = 4 Hz, 4H), 2.21 (m, 4H), 2.04 (s, 6H), 1.72 (s, 14H). HRMS (ESI) calcd for $C_{40}H_{46}ClI_2N_2O_4^+$ 907.1230, found 907.1226.

The Compound F4 was subjected to analogous synthetic steps through cyclization of intermediate Compound G4 to afford a yellow solid in 50% yield, which was the acidic tumor microenvironment activatable near-infrared photosensitizer, designated as LET-I. $^1H$ NMR (500 MHz, $CD_3OD$) $\delta$ 8.52 (d, $J$ = 20 Hz, 1H), 8.40 (d, $J$ = 15 Hz, 1H), 7.93 (d, $J$ = 1.5 Hz, 1H), 7.86 (d, $J$ = 2 Hz, 1H), 7.79 (dd, $J_1$ = 10.5 Hz, $J_2$ = 2 Hz, 1H), 7.74 (dd, $J_1$ = 10.5 Hz, $J_2$ = 2 Hz, 1H), 7.25 (d, $J$ = 10 Hz, 1H), 7.11 (d, $J$ = 10 Hz, 1H), 6.55 (d, $J$ = 20 Hz, 1H), 6.26 (d, $J$ = 15 Hz, 1H), 4.31 (m, 2H), 4.24 (m, 2H), 4.17 (t, $J$ = 5 Hz, 2H), 3.66 (m, 4H), 2.77 (m, 4H), 2.21 (m, 2H), 1.97 (m, 2H), 1.75 (s, 6H), 1.73 (s, 6H). $^{13}C$ NMR (100 MHz, $CDCl_3$) $\delta$ 173.9, 169.9, 167.1, 149.9, 146.7, 142.8, 141.6, 141.4, 140.5, 140.1, 137.2, 136.5, 130.4, 128.7, 126.8, 113.3, 110.2, 104.7, 97.7, 89.9, 85.9, 60.3, 56.9, 47.3, 41.6, 39.8, 27.3, 27.1, 26.9, 25.5, 25.1, 19.9, 19.7. HRMS (ESI) calcd for $C_{36}H_{41}I_2ClN_2O_2^+$ 822.0946, found $[M+H]^+$ 823.1015.

Chemical structural formulas of the near-infrared photosensitizers LET-H, LET-Cl, LET-Br, and LET-I activatable in acidic tumor microenvironment prepared in the Embodiments 1-4 above are shown below:

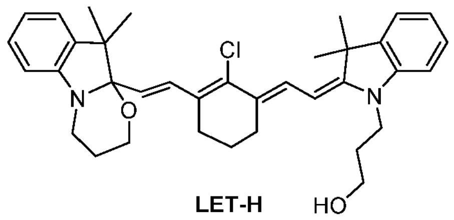
**LET-H**

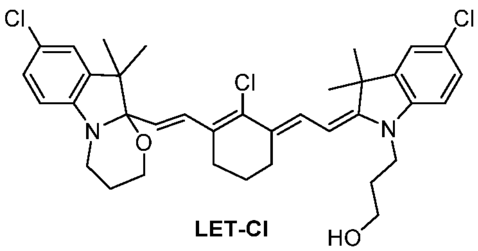
**LET-Cl**

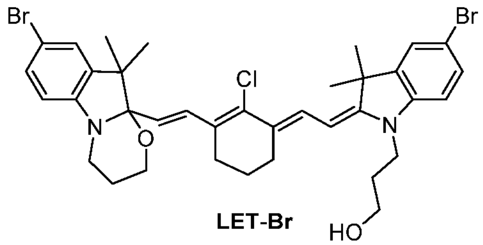
**LET-Br**

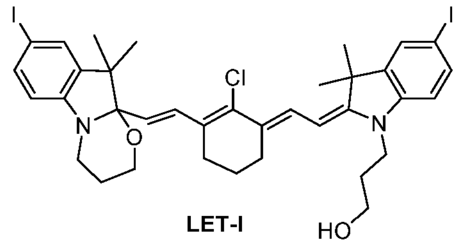
**LET-I**

Embodiment 5

A synthetic route for preparing a near-infrared photosensitizer LET-BCy activatable in acidic tumor microenvironment in the embodiment is shown in FIG. 3, with synthetic steps as follows:

The embodiment provides the acidic tumor microenvironment activatable near-infrared photosensitizer, a synthetic route of which is shown in FIG. 3, with synthetic method including the following steps:

Compound A5 (2 g, 11.8 mmol) and phosphorus pentasulfide (2.25 g, 11.8 mmol) were dissolved in 30 mL pyridine. Under nitrogen atmosphere protection, the reaction mixture was heated to 115°C and refluxed overnight. After completion of the reaction, the reaction mixture was cooled, quenched into 400 mL water, and allowed to cool and stand at 4°C for 3-4 hours. A solid was collected by filtration, washed with 10 mL water three times, and dried under vacuum to afford crude Compound B5 as a yellow solid (1.79 g, 82.1% yield) for direct use in next step.

The Compound B5 (12.4 g, 66.9 mmol) and iodomethane (5.0 mL, 80 mmol) were dissolved in 20 mL acetone, heated to 45 °C under reflux for 30 minutes. A precipitated product was crystallized from methanol to afford Compound C5 as a golden-yellow solid. The precipitated product was unstable and used directly in next step without purification (crude yield: 76%).

A mixture of the crude product Compound C5 (16.4 g, 0.05 mol), Compound D5 (14.4 g, 0.1 mol), and sodium acetate (8.2 g, 0.1 mol) in anhydrous ethanol was reacted at 50 °C for 1 hour. After completion of the reaction, the mixture was cooled and filtered to isolate a residue, which was washed with water and ethanol solution (volume ratio 1:1) to afford Compound E5 (13.8 g, 94% yield). $^1H$ NMR (500MHz, DMSO-$d_6$) $\delta$ 12.78 (s, 1H), 9.41 (d, $J$ = 8.0 Hz, 1H), 8.40 (d, $J$ = 8.0 Hz, 1H), 7.95 (t, $J$ = 8 Hz, 1H), 7.89 (d, $J$ = 8.5 Hz, 1H), 7.79 (d, $J$ = 7.0Hz, 1H), 7.69 (t, $J$ = 8 Hz, 1H), 1.73 (s, 6H).

The Compound E5 (443 mg, 1.5 mmol) and potassium carbonate (622 mg, 4.5 mmol) were mixed in 10 mL N,N-dimethylformamide and stirred at 25 °C for 15 minutes, followed by dropwise addition of the Compound D1 (1.02 g, 4.5 mmol). The reaction mixture was then heated to 100 °C and reacted for 12 hours. The reaction was quenched with 250 mL ice water and extracted with dichloromethane. The crude product was purified by silica gel column chromatography (eluent: dichloromethane/methanol) as eluent to afford Compound F5 as a solid (367 mg, 62% yield). $^1H$ NMR (500 MHz,

$CDCl_3$) δ 8.72 (d, *J* =7.0 Hz, 1H), 8.58 (d, *J* = 7.5 Hz, 1H), 8.48 (d, *J* = 7.0 Hz, 1H), 8.27 (d, *J* = 8.0 Hz, 1H), 8.08 (t, *J* = 7.5 Hz, 1H), 7.95 (t, *J* = 8 Hz, 1H), 5.06 (s, 2H), 3.84 (s, 2H), 3.53 (s, 3H), 2.44 (s, 2H), 1.26 (s, 6H).

The Compound F5 (1.14 g, 2.9 mmol) was dissolved in 4 mL acetic acid and the reaction mixture was refluxed at 90°C for 1 hour. 4 mL concentrated hydrochloric acid was added dropwise to the refluxing mixture until color changed from red to green. The reaction mixture was cooled to room temperature, followed by addition of saturated potassium iodide solution until the product precipitation initiated. The product was collected by filtration, washed with diethyl ether, and dried under vacuum to afford a solid Compound G5 (314.7 mg, 48% yield). $^1H$ NMR (500 MHz, DMSO-$d_6$) δ 9.0 (d, *J* = 7.5 Hz, 1H), 8.81 (d, *J* = 8.0 Hz, 1H), 8.52 (d, *J* = 7.0 Hz, 1H), 8.46 (d, *J* = 8.0 Hz, 1H), 8.150 (t, *J* = 7.5 Hz, 1H), 8.02 (t, *J* = 8 Hz, 1H), 4.75 (t, *J* = 7.0 Hz, 2H), 3.55 (t, *J* = 5.5 Hz, 2H), 3.26 (s, 3H), 2.13 (m, 2H).

The Compound G5 (762.64 mg, 2.19 mmol), the Compound B1 (173 mg, 1 mmol), and sodium acetate (160 mg, 2 mmol) were dissolved in 5 mL acetic anhydride and stirred at 40 °C for 2 hours to synthesize condensation intermediate Compound H5. Subsequently, solid potassium carbonate (164 mg, 1.19 mmol) was added with stirring for 2 hours. The crude product was precipitated with 60 mL diethyl ether and finally purified by column chromatography to afford a solid compound, designated as the near-infrared photosensitizer LET-BCy activatable in acidic tumor microenvironment. HRMS (ESI) calcd for $C_{38}H_{36}ClN_2O_2^+$ 587.2460, found $[M+H]^+$ 587.2459.

A structural formula of the near-infrared photosensitizer LET-BCy activatable in acidic tumor microenvironment prepared in the Embodiment 5 is shown below:

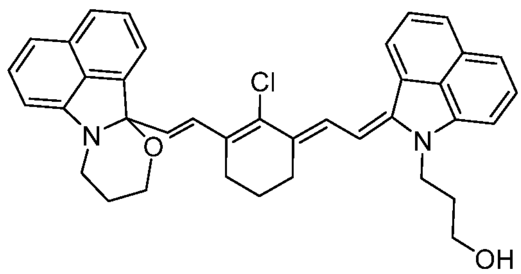

Embodiment 6

A synthetic route for preparing the near-infrared photosensitizer LET-Hcy-N activatable in acidic tumor microenvironment in the embodiment is shown in FIG. 4, with synthetic steps as follows:

Under nitrogen atmosphere protection with ice bath conditions, phosphorus oxychloride (7.7 mL, 82 mmol) was added dropwise to anhydrous N,N-dimethylformamide (7.9 mL, 64 mmol) with stirring for 15 minutes, followed by dropwise addition of the Compound A1 (5 g, 50.9 mmol) to the reaction mixture. The reaction mixture was stirred at room temperature for 2 hours. After completion of the reaction, the reaction mixture was= quenched by pouring into 200 g ice water, and sodium bicarbonate was slowly added to adjust pH to approximately 7. The mixture reaction was then extracted multiple times with ethyl acetate, and organic phases were combined, dried over anhydrous sodium sulfate and concentrated under reduced pressure to afford Compound A6 as an orange oily substance (6.8 g, 93% yield) for direct use in next step without purification.

The Compound A6 (0.97 g, 4 mmol), Compound B6 (0.39 g, 2 mmol), and cesium carbonate (1.95 g, 6 mmol) were dissolved in 20 mL anhydrous N,N-dimethylformamide and stirred at room temperature for 3 days. After completion of the reaction, the reactin mixture was washed with dichloromethane and filtered. A filtrate was collected and washed multiple times with saturated brine, and organic phases were combined, dried over anhydrous sodium sulfate and concentrated under reduced pressure to afford the crude product, which was further purified by silica gel column chromatography (eluent: dichloromethane) to afford Compound C6 as a yellow solid (0.36 g, 64% yield). $^1H$ NMR (400 MHz, $CDCl_3$) δ 10.27 (s, 1H), 7.08 (m, 2H), 6.65 (s, 1H), 6.44 (s, 1H), 3.40 (d, *J* = 6.7 Hz, 4H), 2.59 (m, 2H), 2.45 (t, *J* = 6.0 Hz, 2H), 1.42 (s, 2H), 1.21 (s, 6H).

Under nitrogen atmosphere protection, the Compound C6 (0.28 g, 1.0 mmol), the Compound E1 (0.32 g, 1.2 mmol), potassium carbonate (0.28 g, 2.0 mmol), and 5 mL anhydrous acetic anhydride were added to a reaction flask. The mixture was stirred at room temperature for 24 hours. After completion of the reaction, dichloromethane was added to the reaction mixture, washed with water three times, organic phases were combined, dried over anhydrous sodium sulfate, and concentrated under reduced pressure. A crude product was purified by silica gel column chromatography (eluent: dichloromethane/methanol, volume ratio 20:1) to afford Compound D6 as a dark green solid (0.35 g, 67% yield). $^1H$ NMR (500 MHz, $CDCl_3$) δ 8.50 (d, *J* = 14.1 Hz, 1H), 7.56 (s, 1H), 7.44 (dd, *J* = 26.1, 9.9 Hz, 3H), 7.20 (d, *J* = 8.0 Hz, 1H), 6.82 (d, *J* = 9.0 Hz, 1H), 6.52 (s, 1H), 6.18 (d, *J* = 14.1 Hz, 1H), 4.35 (t, *J* = 6.9 Hz, 2H), 4.21 (t, *J* = 5.9 Hz, 2H), 3.56 (q, *J* = 7.1 Hz, 4H), 3.49 (s, 1H), 2.83 (m, 4H), 2.29 (m, 2H), 1.95 (s, 3H), 1.79 (s, 6H), 1.72 (s, 2H), 1.31 (t, *J* = 7.1 Hz, 6H).

Potassium carbonate (56 mg, 0.4 mmol) was added to a solution of Compound D6 (105 mg, 0.2 mmol) in 2 mL

methanol, and the reaction mixture was stirred at room temperature overnight. The compound D6 underwent a subsequent cyclization reaction through intermediate compound E6. After completion of the reaction, the reaction mixture was extracted with dichloromethane/saturated aqueous sodium bicarbonate, organic phases were combined, dried over anhydrous sodium sulfate, and concentrated under reduced pressure. The crude product was purified by basic alumina column chromatography (eluent: petroleum ether and dichloromethane, volume ratio 50:1) to afford a yellow solid, which was the acidic tumor microenvironment activatable near-infrared photosensitizer, designated as LET-Hcy-N (86 mg, 89% yield). $^1$H NMR (500 MHz, MeOD) δ 8.65 (d, *J* = 12.6 Hz, 1H), 7.55 (d, *J* = 8.0 Hz, 2H), 7.48 (m, 2H), 7.39 (d, *J* = 7.9 Hz, 1H), 7.31 (td, *J* = 7.4, 1.0 Hz, 1H), 6.93 (dd, *J* = 9.0, 2.4 Hz, 1H), 6.73 (d, *J* = 2.5 Hz, 1H), 6.38 (d, *J* = 12.7 Hz, 1H), 4.29 (t, *J* = 7.2 Hz, 2H), 3.69 (t, *J* = 5.7 Hz, 2H), 3.60 (q, *J* = 7.1 Hz, 4H), 2.76 (dd, *J* = 26.1, 6.3 Hz, 4H), 2.08 (m, 2H), 1.94 (t, *J* = 5.2 Hz, 2H), 1.80 (s, 6H), 1.28 (d, *J* = 7.1 Hz, 6H)。HRMS (ESI) calcd for $C_{32}H_{38}N_2O_2^+$ 483.293, found $[M+H]^+$ 483.765.

A structural formula of the near-infrared photosensitizer LET-Hcy-N activatable in acidic tumor microenvironment prepared in the Embodiment is shown below:

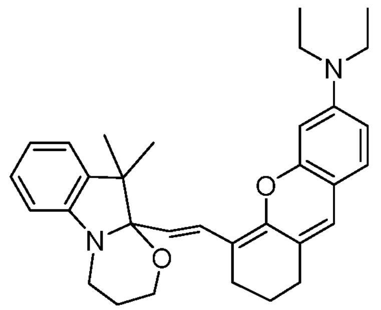

Embodiment 7

A synthetic route for preparing a near-infrared photosensitizer LET-BHcy-ROS activatable in acidic tumor microenvironment in the embodiment is shown in FIG. 5, with synthetic steps as follows:

The Compound A6 (1 g, 6.94 mmol), Compound A7 (0.88 g, 5.78 mmol), and cesium carbonate (5.65 g, 17.35 mmol) were dissolved in 40 mL N,N-dimethylformamide and stirred at room temperature for 3 days. After completion of the reaction, the reaction mixture was filtered, and the filtrate was concentrated under vacuum. The residue was dissolved in 50 mL dichloromethane and washed with distilled water (3 × 25 mL), organic phases were combined, dried over anhydrous magnesium sulfate, filtered, and concentrated under vacuum. Purification by column chromatography (eluent: n-hexane and ethyl acetate, volume ratio 1:3) to afford Compound B7 as a yellow solid (1.09 g, 78% yield) for direct use in next step.

The Compound B7 (1 g, 2.19 mmol) was dissolved in anhydrous dichloromethane under nitrogen atmosphere protection. The reaction mixture was cooled to 0 °C in an ice bath, boron tribromide (2.2 mL, 22.6 mmol) was added dropwise, and the reaction mixture was stirred at room temperature overnight. The reaction mixture was quenched with water and extracted with dichloromethane. Organic phase was washed with water three times, dried over anhydrous magnesium sulfate, filtered, and concentrated under vacuum. The residue was purified by silica gel column chromatography (eluent: dichloromethane and methanol, volume ratio 20:1) to afford Compound C7 as a brown solid (0.34 g, 34% yield). $^1$H NMR (500 MHz, DMSO-*d*$_6$): δ 10.20 (s, 2H), 7.19 (d, *J* = 8.5 Hz, 1H), 6.93 (s, 1H), 6.63 (d, *J* = 2.0 Hz, 1H), 6.60 (dd, *J* = 8.5, 2.5 Hz, 1H), 2.55 (m, 2H), 2.29 (t, *J* = 6 Hz, 2H), 1.64 (m, 2H).

To a solution of the Compound C7 (114.1 mg, 0.5 mmol) in dichloromethane was added Compound D7 (297 mg, 1.0 mmol) and potassium carbonate (138.21 mg, 1.0 mmol). The reaction mixture was stirred at room temperature overnight. After completion of the reaction, excess potassium carbonate was removed by filtration. The filtrate was dried over anhydrous sodium sulfate and concentrated. The crude product was purified by silica gel column chromatography (eluent: dichloromethane and methanol, 30:1 volume ratio) to afford Compound E7 as a yellow solid (193 mg, 53% yield). HRMS (ESI) calcd for $C_{27}H_{30}BO_5^+$ 445.2108, found $[M+H]^+$ 445.217. Under nitrogen atmosphere protection, the Compound G5 (68.9 mg, 0.2 mmol) and the Compound E7 (50 mg, 0.1 mmol) were dissolved in 10 mL anhydrous ethanol and heated to 80 °C with stirring for 12 hours to form condensation intermediate Compound F7. Subsequently, solid potassium carbonate (16.5 mg, 0.12 mmol) was added, and the reaction mixture was stirred for an additional 2 hours. The crude product was precipitated with 30 mL diethyl ether, concentrated, and purified by column chromatography (eluent: dichloromethane and methanol, volume ratio 20:1), and the solvent was evaporated to afford a solid compound (45 mg, 55.4% yield), which was the acidic tumor microenvironment activatable near-infrared photosensitizer, designated as LET-BHcy-ROS. $^1$H NMR (500 MHz, MeOD) δ 8.89 (d, *J* = 14.5 Hz, 1H), 8.52 (d, *J* = 7.5 Hz, 1H), 8.15 (d, *J* = 4.0 Hz, 1H), 7.96 (t, *J* = 7.5 Hz, 1H), 7.73 (dd, *J* = 8.0, 4.5 Hz, 3H), 7.59 (m, 2H), 7.41 (m, 4H), 7.16 (d, *J* = 4.0 Hz, 1H), 7.02 (dd, *J* = 8.5, 2.0 Hz, 1H), 6.76 (d, *J* = 14.5 Hz, 1H), 5.24 (s, 2H), 4.42 (t, *J* = 7.0 Hz, 2H), 3.65 (t, *J* = 6.0 Hz, 2H), 2.70 (m, 4H), 2.04 (m, 2H),

1.88 (m, 2H), 1.33 (s, 12H).

Embodiment 8

A synthetic route for preparing a near-infrared photosensitizer LET-BHcy-GSH activatable in acidic tumor microenvironment in the embodiment is shown in FIG. 5, with synthetic steps as follows:
Under nitrogen atmosphere protection with ice bath condition at 0 °C, Compound D8 (0.66 g, 2.5 mmol) and triethylamine (0.26 mL, 3 mmol) were sequentially added to a solution of the Compound C7 (114.1 mg, 0.5 mmol) in anhydrous dichloromethane. The reaction mixture was stirred at room temperature overnight. After completion of the reaction, the reaction mixture was washed with saturated sodium chloride solution and distilled water, dried over anhydrous sodium sulfate, and concentrated. Purification by column chromatography to afford Compound E8 as a yellow solid (106 mg, 48% yield). HRMS (ESI) calcd for $C_{20}H_{15}N_2SO_9^+$ 459.0420, found $[M+H]^+$ 459.067.

Under nitrogen atmosphere protection, Compound G5 (66.8 mg, 0.2 mmol) and the Compound E8 (50 mg, 0.1 mmol) were dissolved in 10 mL anhydrous ethanol. The reaction mixture was heated to 80 °C and stirred overnight to form condensation intermediate Compound F8. Subsequently, solid potassium carbonate (16.5 mg, 0.12 mmol) was added, and the mixture was stirred for 2 hours. The crude product was precipitated with 30 mL diethyl ether, concentrated under reduced pressure, and purified by column chromatography (eluent: dichloromethane and methanol, volume ratio 20:1) to afford the final product as a solid compound (34 mg, 45.9% yield), which was the acidic tumor microenvironment activatable near-infrared photosensitizer, designated as LET-BHcy-GSH. $^1$H NMR (500 MHz, MeOD) δ 9.15 (d, *J* = 15 Hz, 1H), 8.96 (d, *J* = 5 Hz, 1H), 8.74 (d, *J* = 10 Hz, 1H), 8.52 (dd, *J* = 10, 5 Hz, 1H), 8.37 (d, *J* = 5 Hz, 1H), 8.00 (m, 2H), 7.84 (d, *J* = 10 Hz, 1H), 7.60 (d, *J* = 5 Hz, 1H), 7.43 (d, *J* = 5 Hz, 1H), 7.37 (s, 1H), 7.14 (dd, *J* = 10, 5 Hz, 1H), 7.03 (d, *J* = 15 Hz, 1H), 4.60 (t, *J* = 5 Hz, 2H), 3.67 (t, *J* = 5 Hz, 2H), 2.82 (m, 4H), 2.14 (m, 2H), 1.97 (m, 2H).

Structural formulas of the near-infrared photosensitizer LET-BHcy-ROS and LET-BHcy-GSH activatable in acidic tumor microenvironment prepared in the Embodiments 7-8 are shown below:

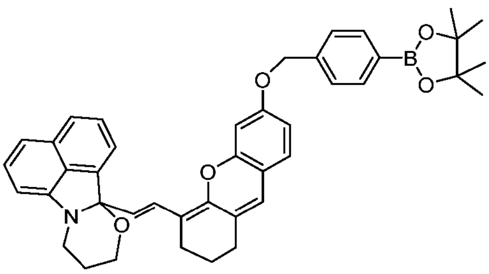

**LET-BHcy-ROS**

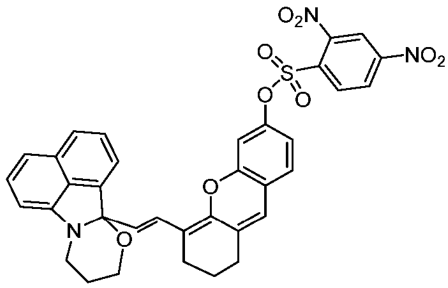

**LET-BHcy-GSH**

Performance Testing

(1) Spectral characterization

The photosensitizers prepared in the Embodiments 1-8 were individually dissolved in dimethyl sulfoxide to prepare 10 mM photosensitizer stock solution, which were stored at 4 °C for subsequent use. All experiments were conducted in PBS buffer (1 ×) containing 1% DMSO. 2 μL of the photosensitizer stock solution was added to 2 mL of PBS buffer solutions at different pH values. Absorption spectra and fluorescence spectra of each photosensitizer under various pH conditions were recorded using ultraviolet-visible spectrophotometry and fluorescence spectrophotometry.

Absorption test results of each photosensitizer are shown in FIG. 6. Since all photosensitizer molecules share the same activation mechanism (as shown in FIG. 1) involving cleavage of indole ortho-ether oxygen bond, ultraviolet-visible absorption spectra exhibit similar pH-dependent trends. As solution pH decreases, absorbance in the near-infrared region progressively increases while the visible light absorbance gradually decreases. For fluorescence emission, representative photosensitizers LET-H, LET-I, and LET-Hcy-N were examined, with the fluorescence spectra depicted in panels (a), (b), (c), (d), and (e) of FIG. 7. In the visible light region, the fluorescence intensity of LET-H and LET-I decreased with increasing pH. Conversely, in the near-infrared region, LET-H, LET-I, and LET-Hcy-N demonstrated enhanced fluorescence emission intensity at higher pH values. The results confirm that the photosensitizers of the present disclosure are activatable in mild acidic environment, exhibiting both near-infrared absorption and fluorescence emission.

(2) Singlet oxygen generation assay

The photosensitizers LET-R (R = H, Cl, Br, I) prepared in the Embodiments 1-4 were assessed. 1,3-Diphenylisobenzofuran (DPBF) was selected as a singlet oxygen trapping agent to determine a singlet oxygen generation capability of each photosensitizer molecule.

A 1 mM DPBF stock solution was first prepared for use. An appropriate volume of freshly prepared DPBF solution was then added to DMSO to adjust the absorbance at 415 nm to approximately 1.0, followed by addition of photosensitizer solution at appropriate concentration. The ultraviolet-visible absorption spectra were measured after different irradiation times with a near-infrared laser. The singlet oxygen generation capability of each photosensitizer was evaluated by monitoring the absorbance changes of DPBF at 415 nm.

The test results are shown in panel (a) of FIG. 8. Compared to the non-activated photosensitizer molecules LET-R (R = H, Cl, Br, I), the activated photosensitizers LET-R' (R = H, Cl, Br, I) demonstrated singlet oxygen generation capability, indicating that the photosensitizers prepared in the Embodiments 1-4 of the present disclosure can produce near-infrared photodynamic therapeutic effects upon activation. Furthermore, LET-I' exhibited superior singlet oxygen generation efficiency, demonstrating that halogen atom modification of the photosensitizer molecular framework can enhance singlet oxygen production capacity.

Using indocyanine green (ICG) as a reference standard, the singlet oxygen quantum yields of photosensitizers LET-R (R = H, Cl, Br, I) were determined. At first, an appropriate volume of freshly prepared DPBF solution was added to dichloromethane to adjust the absorbance at 415 nm to approximately 1.0, followed by addition of test solution (photosensitizer or ICG) at optimized concentration. Samples were then irradiated with a near-infrared laser, and time-dependent ultraviolet-visible spectral changes were recorded. The singlet oxygen quantum yield was calculated using Formula (1):

$$\Phi_{\Delta}=\Phi_{MB}*(k_{ps}*F_{MB})/(k_{MB}*F_{ps}) \quad (1)$$

$\Phi_{\Delta}$ represents the singlet oxygen quantum yield of test molecules, and $\Phi_{ICG}$ represents the singlet oxygen quantum yield of ICG. "ps" represents the photosensitizer under investigation, while k represents a slope of DPBF absorbance decay at 415 nm over time. And correction factor F is calculated using Formula (2):

$$F=1-10^{OD} \quad (2)$$

In Formula (2), OD represents the absorbance of solutions at a laser wavelength.

The test results are shown in panel (b) of FIG. 8, which quantitatively confirms that the photosensitizer LET-I' exhibits superior singlet oxygen generation capability.

Singlet oxygen generation and characterization were further analyzed by electron spin resonance (ESR) spectroscopy. Using photosensitizers LET-H' and LET-I' as examples, 2,2,6,6-tetramethyl-4-piperidone hydrochloride (TEMP) was employed as the singlet oxygen trapping agent. Two groups of photosensitizer stock solutions were prepared and diluted with TEMP added, one group remained untreated, while another was irradiated with the near-infrared laser, and a blank control group was also prepared for comparison.

The experimental results are shown in panel (c) of FIG. 8, reveal that near-infrared irradiation of samples containing activated photosensitizers LET-H' or LET-I' generated three isointense paramagnetic resonance peaks (1:1:1) in the EPR spectrum, confirming singlet oxygen production by both LET-H' and LET-I'.

(3) Evaluation of therapeutic efficacy at cellular level

The photodynamic therapeutic efficacy was evaluated using standardized MTT assay protocols to assess the effects of photosensitizer molecules on viability of murine mammary carcinoma cells (4T1 cell line).

Cell culture was performed using Dulbecco's Modified Eagle Medium (DMEM) high glucose supplemented with 10% fetal bovine serum (FBS) and 1% antibiotic-antimycotic solution (penicillin-streptomycin mixture), and maintained in a humidified incubator at 37 °C with 5% $CO_2$. Experimental procedures were initiated when cell density reached 80% confluency. Using photosensitizers LET-H and LET-I as examples, 4T1 cells were seeded in 96-well plates (100 $\mu$L, $5\times10^3$ cells/well) and cultured for 24 hours, then original medium was discarded and replaced with medium containing photosensitizer molecules at a concentration of 20 $\mu$M. After 6 hours of incubation, the medium in each well was replaced with fresh medium, and irradiated with an 808 nm (0.2 W/$cm^2$) laser for different times (0, 5, 10 and 20 min), and placed back in cell culture incubator for continued incubation. After irradiation, the plates were returned to the incubator for

continued culture. After 12 hours of incubation, 10 μL of CCK-8 solution was added to each well, and the plates were incubated for one additional hour. Absorbance was subsequently measured at 450 nm using a microplate reader.

The test results are shown in FIG. 9, LET-I group exhibited below 30% cell viability after 20 minutes laser irradiation, which was significantly lower than the cell viability of LET-H group after the same irradiation duration, thereby conclusively establishing superior phototoxic potency of the LET-I.

(4) Evaluation of visualized photodynamic therapy efficacy at in vivo level

Construction of a murine tumor model for visualized photodynamic therapy evaluation.

Female BALB/c nude mice (6 weeks old) were purchased for establishing a murine tumor model. 4T1 cells in logarithmic growth phase were trypsinized and resuspended in serum-free medium, maintaining cell suspension on ice prior to inoculation. All mice were randomly divided into groups of 5 mice each. A 100 μL suspension of 4T1 cells was subcutaneously injected into right posterior axillary region of each mouse. When tumor volumes reached 80 $mm^3$, photosensitizer molecules were administered via intratumoral injection or tail vein injection. Using small animal fluorescence imaging system (IVIS Spectrum) and small animal photoacoustic imaging system (VisualSonics Vevo LAZR system), fluorescence signals and photoacoustic signals in tumor area were detected over time. During imaging procedures, all mice were anesthetized with isoflurane (2% in oxygen). Using LET-H and LET-I as examples, to enhance biocompatibility, the photosensitizer molecules were nanoengineered with folate-modified amphiphilic polymers, designated as LET-H-FA and LET-I-FA, respectively.

As shown in panels (a) and (b) of FIG. 10, after intratumoral injection of LET-I-FA, a fluorescence signal in tumor gradually increased, while the fluorescence intensity remained essentially unchanged when subcutaneously injected into normal tissue (leg muscle). At 20 minutes post-injection, the tumor fluorescence intensity in mouse tumors demonstrated significant enhancement compared to normal tissues. Meanwhile, PBS control group shows lower and unchanged fluorescence signals in both tumors and normal tissues, demonstrating that LET-I-FA can be specifically activated in tumors and generate strong fluorescence signals. As shown further in panels (c) and (d) of FIG. 10, following either intratumoral or subcutaneous administration of LET-I-FA, photoacoustic signal intensity in tumors reached maximum levels at 20 minutes post-injection, while the photoacoustic signal in normal tissues maintained essentially unchanged and consistently lower than in tumor signal intensity. These results further demonstrate that LET-I-FA can be specifically activated in tumors and generate strong photoacoustic signals.

Tumor-bearing mice were randomly divided into seven groups: (1) PBS group; (2) light irradiation only group; (3) LET-I-FA group; (4) LET-H-FA group; (5) LET-I + light irradiation group; (6) LET-H-FA + light irradiation group; and (7) LET-I-FA + light irradiation group. Each group of photosensitizer solutions was injected into mice via tail vein injection at a dose of 10 μmol/kg, with PBS administered at 100 μL per mouse. At 12 hours post-injection, groups requiring laser irradiation were exposed to 808 nm laser irradiation at 0.2 $W/cm^2$ power density for 30 minutes per mouse. Body weight changes were observed every other day for each group of mice from initial administration. As shown in panel (a) of FIG. 11, the results demonstrate that body weights of mice showed no significant changes during treatment course, indicating excellent biosafety of the photosensitizer molecules used.

After 14 days of treatment, all mice were euthanized, and tumors were excised for gravimetric analysis and photographic documentation. As shown in panels (b) and (c) of FIG. 11, the LET-I-FA + light irradiation group exhibited significant tumor suppression, demonstrating superior therapeutic efficacy compared to the LET-H-FA + light irradiation group.

Furthermore, upon dissection, major organs (heart, liver, spleen, lungs, and kidneys) were fixed in paraformaldehyde, processed for hematoxylin and eosin (H&E) staining, and examined histologically. As shown in panel (a) of FIG. 12, the results indicate that no obvious changes occurred in major organs after photosensitizer treatment.

Three additional groups of mice with three mice per group, photosensitizer solutions LET-H-FA or LET-I-FA were injected into mice via tail vein injection at a dose of 10 μmol/kg, with PBS administered at 100 μL per mouse. After 14 days, blood samples were collected from each group of mice by orbital puncture, and centrifuged to obtain serum for biochemical analysis of the mice, to evaluate impact on hepatic and renal function in mice. As shown in panel (b) of FIG. 12, no significant abnormalities were observed in blood biochemical parameters across all groups, further confirming favorable biosafety of the photosensitizer molecules.

In summary, the near-infrared photosensitizers activatable in acidic tumor microenvironment provided by the present disclosure enable fluorescence imaging/photoacoustic imaging-guided efficient and precise photodynamic tumor therapy. By incorporating pH-response groups, the near-infrared photosensitizers exhibit enhanced tumor selectivity, thereby significantly reducing phototoxicity to normal tissues. Simultaneously, the tumor-specific activation characteristic confers the photosensitizer molecules with higher imaging signal-to-noise ratio, facilitating precise visualization during photodynamic therapy. Furthermore, through simple modification of halogen substituents on molecular scaffold, the singlet oxygen generation capability of the photosensitizer molecules can be enhanced, effectively inhibiting tumor growth. This approach establishes novel insights for developing more novel near-infrared photosensitizers.

It should be understood that the application of the present disclosure is not limited to the examples described above. For those skilled in the art, various modifications and variations can be made based on the above description, and all such modifications and variations should fall within the protection scope of the appended claims in the present disclosure.

## Claims

1. An acidic tumor microenvironment activatable near-infrared photosensitizer, comprising a chemical structure formula represented by any one of formulas (I) to (VI):

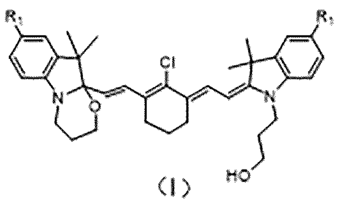
(I)

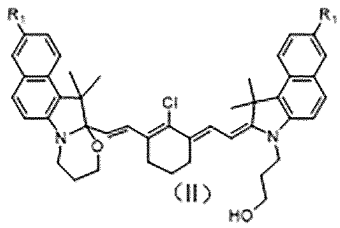
(II)

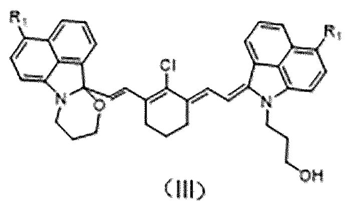
(III)

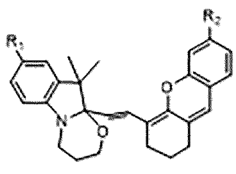
(IV)

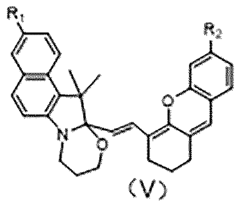
(V)

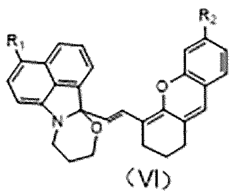
(VI)

;

wherein $R_1$ is a hydrogen, fluorine, chlorine, bromine, or iodine atom; $R_2$ is -OH, $-NH_2$,

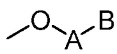 , 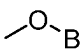 or 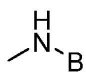 ,

A is a self-eliminating group; B is a biomarker-responsive group.

2. The acidic tumor microenvironment activatable near-infrared photosensitizer according to claim 1, wherein the self-eliminating group A is

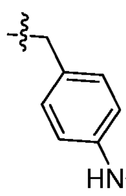 ,

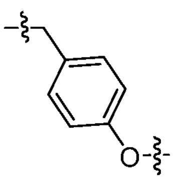 , or 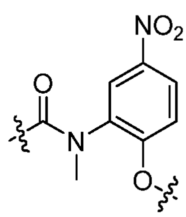 .

3. The acidic tumor microenvironment activatable near-infrared photosensitizer according to claim 1, wherein the biomarker-responsive group B is

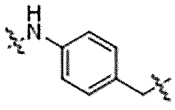, 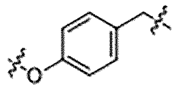, 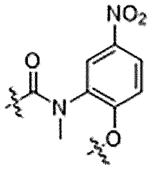, 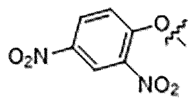,

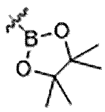, 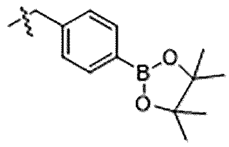, 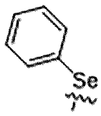, 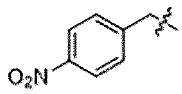, 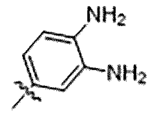,

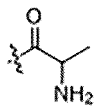, 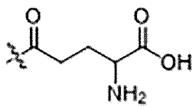, 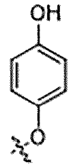, 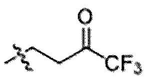, 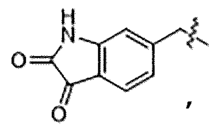,

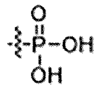, 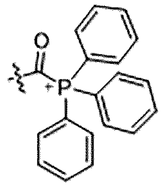, 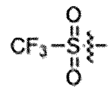, 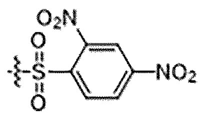, 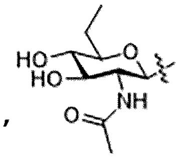,

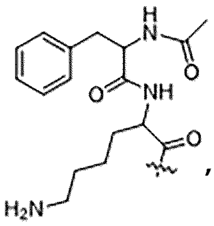, 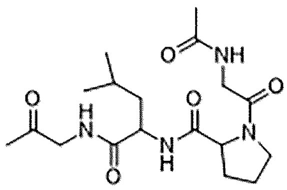 or 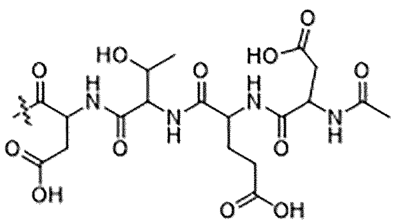.

4. The acidic tumor microenvironment activatable near-infrared photosensitizer according to claim 1, wherein the near-infrared photosensitizers represented by formulas (I) to (VI), upon activation in an acidic environment, form corresponding structures in sequence represented by formulas (I') to (VI'):

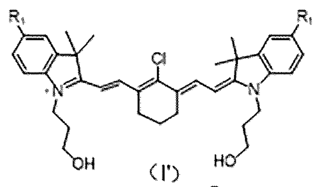
(I')

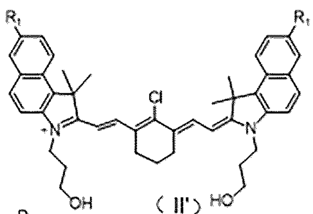
(II')

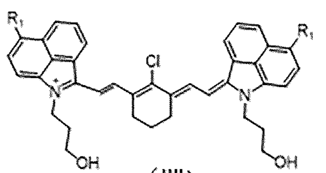
(III')

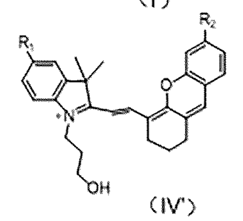
(IV')

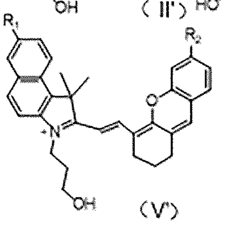
(V')

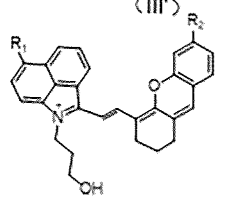
(VI').

5. The acidic tumor microenvironment activatable near-infrared photosensitizer according to claim 1, comprising following chemical structural formulas:

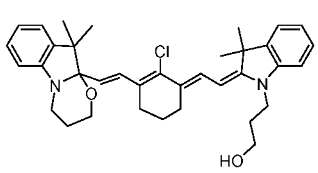
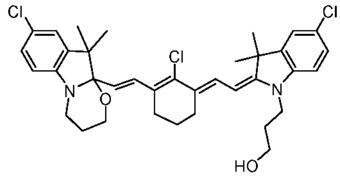
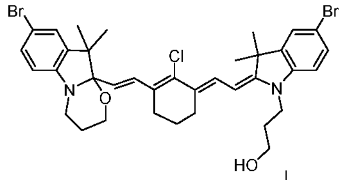
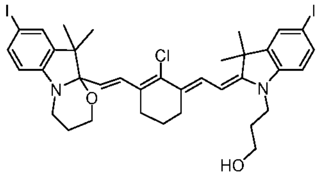
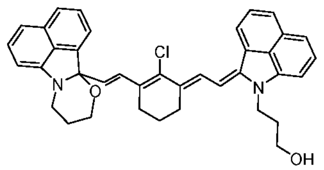
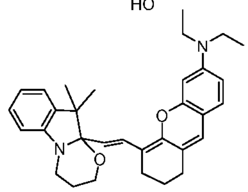
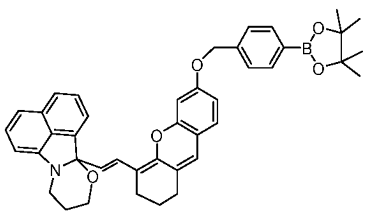
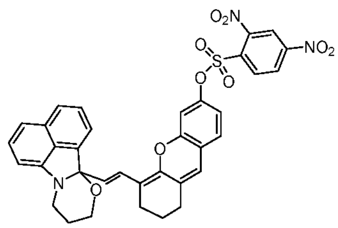

**6.** A method for preparing the acidic tumor microenvironment activatable near-infrared photosensitizer according to any one of claims 1 to 5, comprising steps of:

S1, performing a condensation reaction between compound 1 represented by formula 1 and compound 4 represented by formula 4 to obtain compound 6 represented by formula 6;
or performing the condensation reaction between compound 2 represented by formula 2 and the compound 4 represented by the formula 4 obtain compound 7 represented by formula 7;
or performing the condensation reaction between compound 3 represented by formula 3 and the compound 4 represented by the formula 4 to obtain compound 8 represented by formula 8;
or performing the condensation reaction between the compound 1 represented by the formula 1 and compound 5 represented by formula 5 to obtain compound 9 represented by formula 9;
or performing the condensation reaction between the compound 2 represented by the formula 2 and the compound 5 represented by the formula 5 to obtain compound 10 represented by formula 10;
or performing the condensation reaction between the compound 3 represented by the formula 3 and the compound 5 represented by the formula 5 to obtain compound 11 represented by formula 11; and,
S2. subjecting the compound 6 to sequential ester hydrolysis reaction and ring-closure reaction under alkaline conditions to obtain the acidic tumor microenvironment activatable near-infrared photosensitizer represented by formula (I);
or subjecting the compound 7 to sequential the ester hydrolysis reaction and the ring-closure reaction under the alkaline conditions to obtain the acidic tumor microenvironment activatable near-infrared photosensitizer represented by formula (II);
or subjecting the compound 8 to the ring-closure reaction under the alkaline conditions to obtain the acidic tumor microenvironment activatable near-infrared photosensitizer represented by formula (III);
or subjecting the compound 9 to sequential the ester hydrolysis reaction and the ring-closure reaction under the alkaline conditions to obtain the acidic tumor microenvironment activatable near-infrared photosensitizer represented by formula (IV);
or subjecting the compound 10 to sequential the ester hydrolysis reaction and the ring-closure reaction under the alkaline conditions to obtain the acidic tumor microenvironment activatable near-infrared photosensitizer represented by formula (V);
or subjecting the compound 11 to the ring-closure reaction under the alkaline conditions to obtain the acidic tumor microenvironment activatable near-infrared photosensitizer represented by formula (VI);
wherein chemical structural formulas of the compound 1 to the compound 11 are represented by the formula 1 to the formula 11 as shown below:

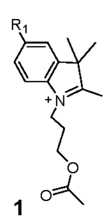
1
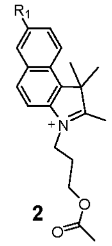
2
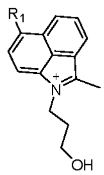
3
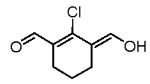
4
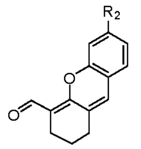
5

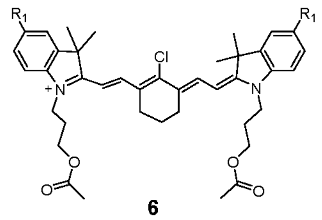
6
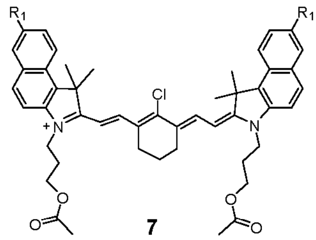
7
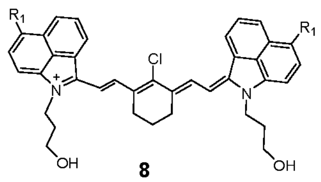
8

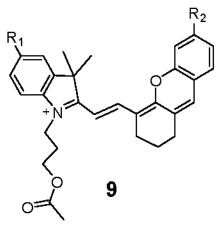
9
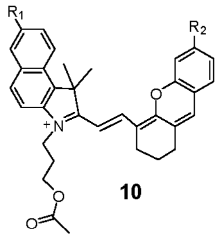
10
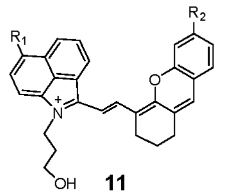
11

;

a temperature of the condensation reaction in the step S1 is 50 °C to 100 °C and a duration of the condensation reaction is 2 to 12 hours;

the alkaline condition required for the ring-closure reaction in the step S2 is one or more of potassium carbonate, cesium carbonate, potassium hydroxide, sodium hydroxide, sodium hydride, aqueous ammonia, and triethylamine; and/or, a temperature of the ring-closure reaction is 0 to 50 °C, and a duration of the ring-closure reaction is 2 to 12 hours.

**7.** The method for preparing the acidic tumor microenvironment activatable near-infrared photosensitizer according to claim 6, wherein the compound 1 in the step S1 is prepared by a substitution reaction between the compound 12 and the compound 13;

wherein a temperature of the substitution reaction is 50 °C to 150 °C, and a duration of the substitution reaction is 24 to 72 hours; and/or, a solvent employed in the substitution reaction is one or more of acetonitrile, N,N-dimethylformamide, dichloromethane, chloroform, toluene, and o-dichlorobenzene;

chemical structural formulas of the compound 12 and the compound 13 are as shown below:

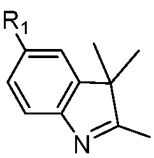

**12**

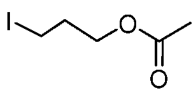

**13**

.

**8.** The method for preparing the acidic tumor microenvironment activatable near-infrared photosensitizer according to claim 6, wherein the compound 3 in the step S1 is prepared through following steps:

subjecting compound 14 to a sulfuration reaction to obtain compound 15;

subjecting the compound 15 to an iodination reaction to obtain compound 16;

reacting the compound 16 with compound 17 in the substitution reaction to obtain compound 18;
reacting the compound 18 with the compound 13 in the substitution reaction to obtain compound 19;
subjecting the compound 19 to a hydrolysis reaction to obtain the compound 3;
chemical structural formulas of the compounds 13 to 19 are as shown below:

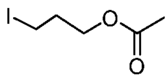
13

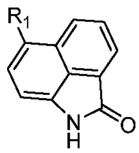
14

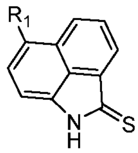
15

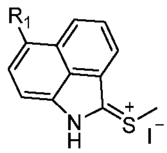
16

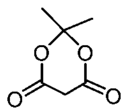
17

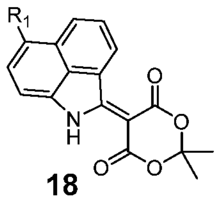
18

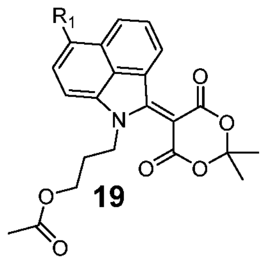
19

.

**9.** An application of the acidic tumor microenvironment activatable near-infrared photosensitizer according to any one of claims 1 to 5, wherein the photosensitizer is for preparing a tumor diagnostic agent and/or a tumor therapeutic agent.

**10.** The application of the acidic tumor microenvironment activatable near-infrared photosensitizer according to claim 9, wherein the tumor diagnostic agent comprises a fluorescence imaging agent or a photoacoustic imaging agent; and/or the tumor therapeutic agent comprises a photodynamic therapy agent or a combination therapy agent for photodynamic therapy with other treatment regimens.

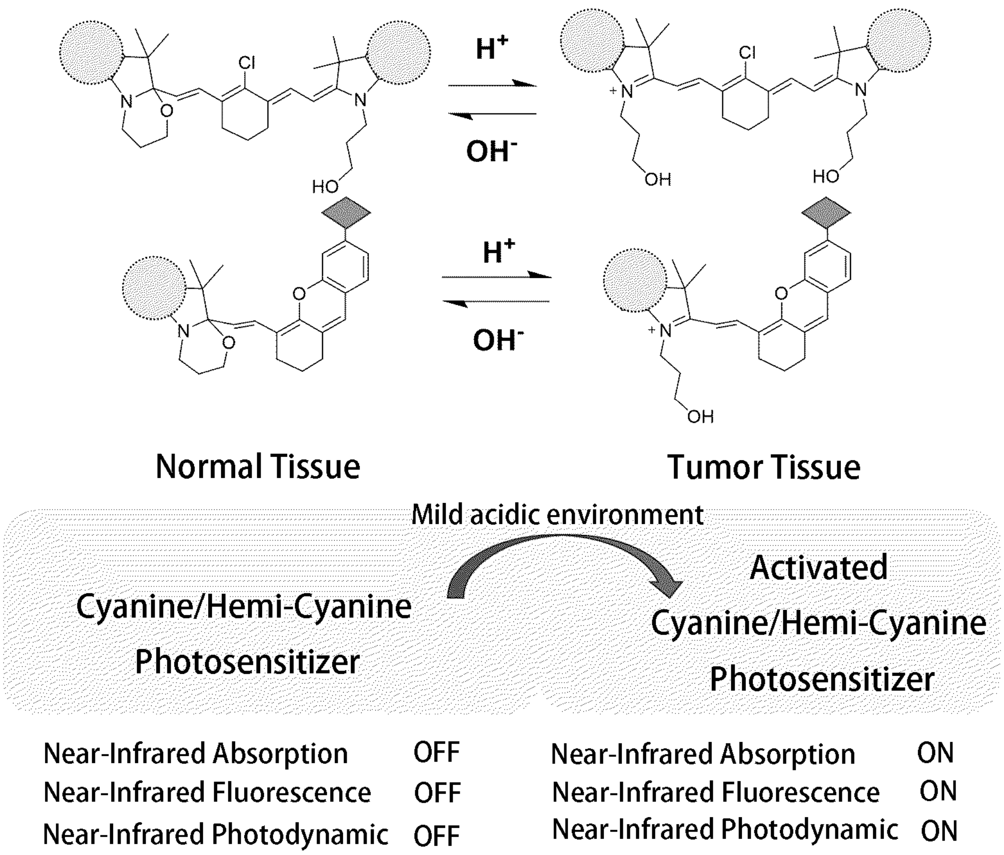
H+
OH-
H+
OH-
Normal Tissue
Tumor Tissue
Mild acidic environment
Cyanine/Hemi-Cyanine Photosensitizer
Activated Cyanine/Hemi-Cyanine Photosensitizer
Near-Infrared Absorption OFF
Near-Infrared Fluorescence OFF
Near-Infrared Photodynamic OFF
Near-Infrared Absorption ON
Near-Infrared Fluorescence ON
Near-Infrared Photodynamic ON

FIG. 1

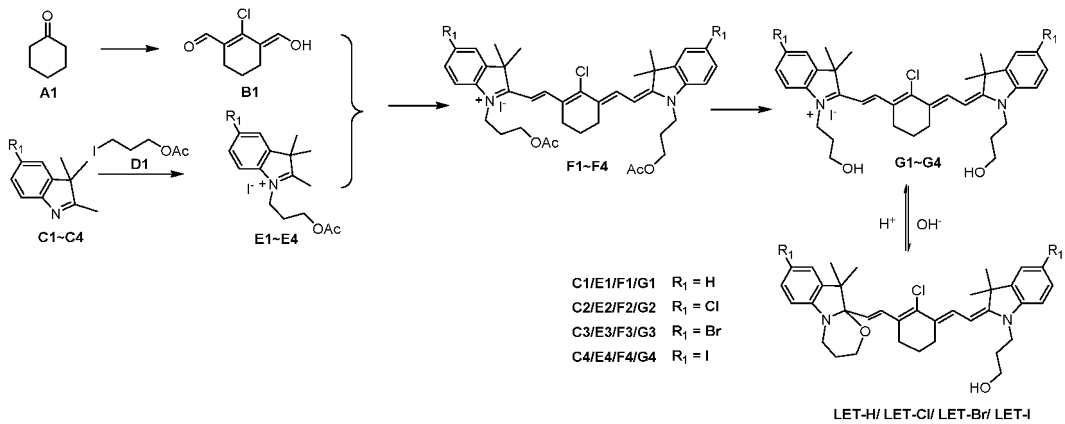
A1
B1
D1
C1~C4
E1~E4
F1~F4
G1~G4
H+
OH-
C1/E1/F1/G1 $R_1$ = H
C2/E2/F2/G2 $R_1$ = Cl
C3/E3/F3/G3 $R_1$ = Br
C4/E4/F4/G4 $R_1$ = I
LET-H/ LET-Cl/ LET-Br/ LET-I

FIG. 2

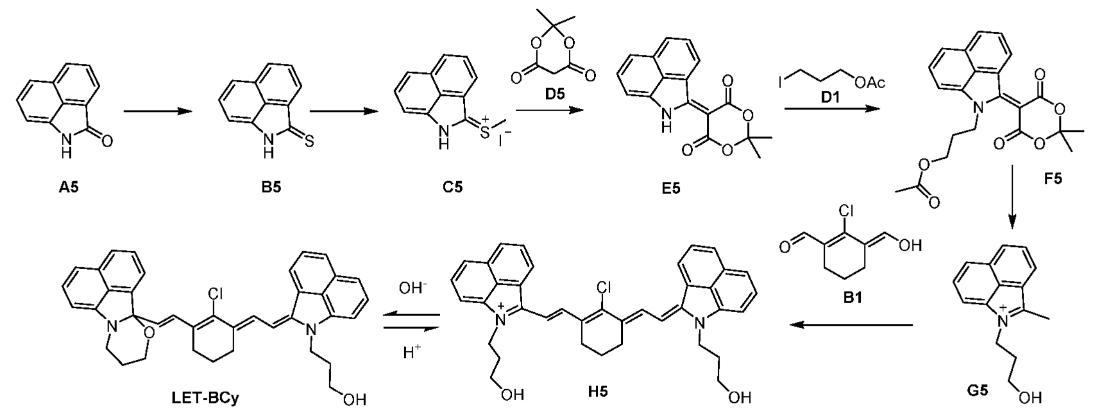

A5 B5 C5 E5 F5 G5 H5 LET-BCy

FIG. 3

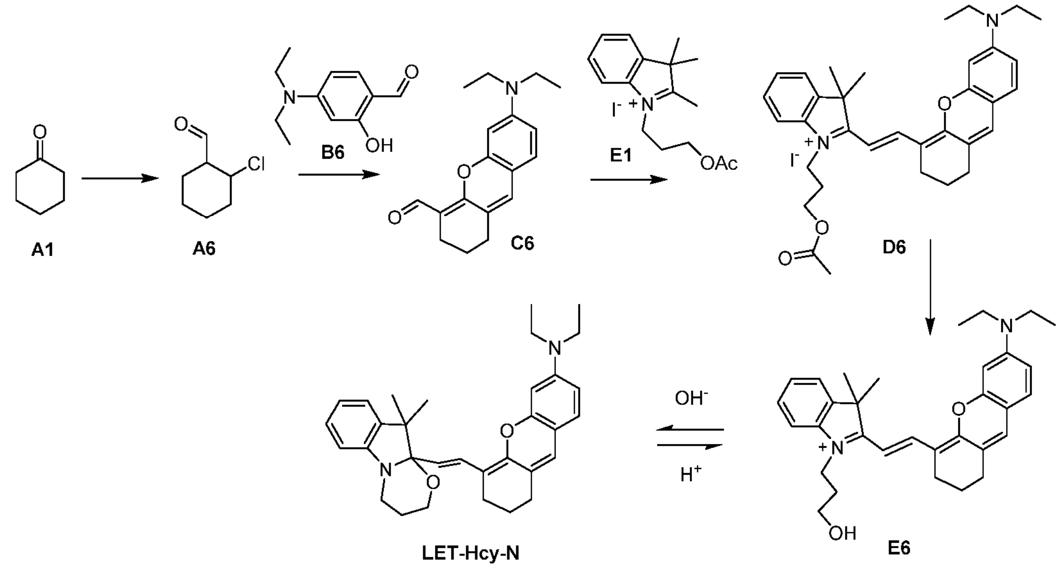

A1 A6 C6 D6 E6 LET-Hcy-N

FIG. 4

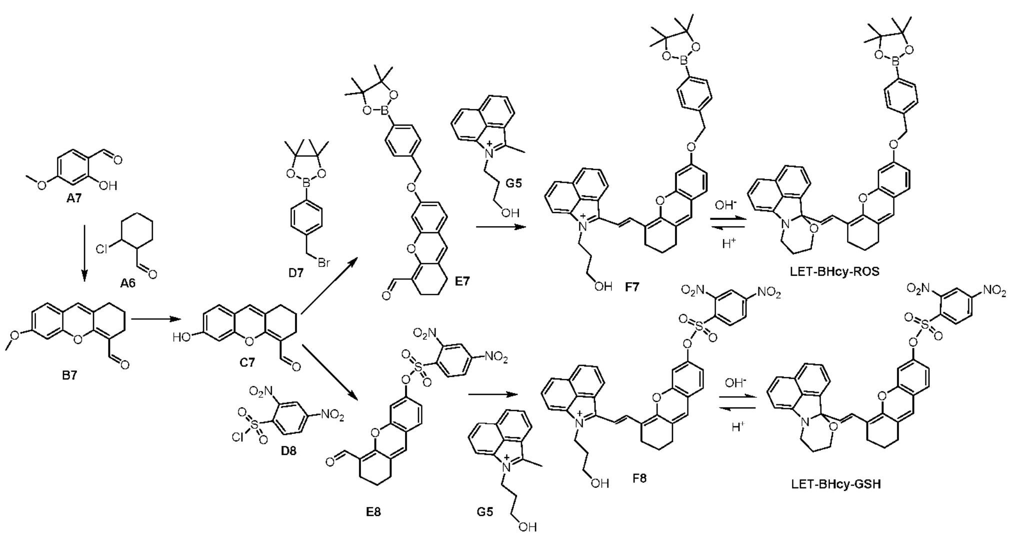


FIG. 5

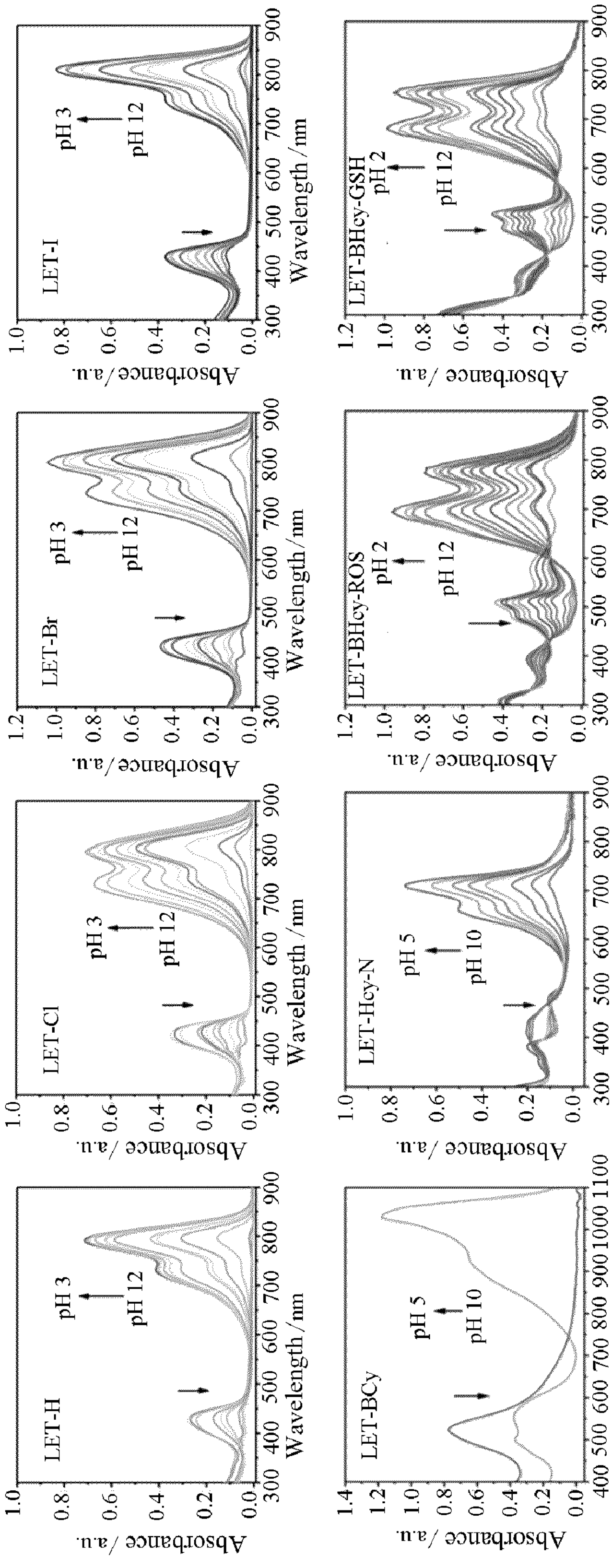
LET-H
pH 3
pH 12
LET-Cl
pH 3
pH 12
LET-Br
pH 3
pH 12
LET-I
pH 3
pH 12
LET-BCy
pH 5
pH 10
LET-Hcy-N
pH 5
pH 10
LET-BHcy-ROS
pH 2
pH 12
LET-BHcy-GSH
pH 2
pH 12
Absorbance /a.u.
Wavelength /nm

FIG. 6

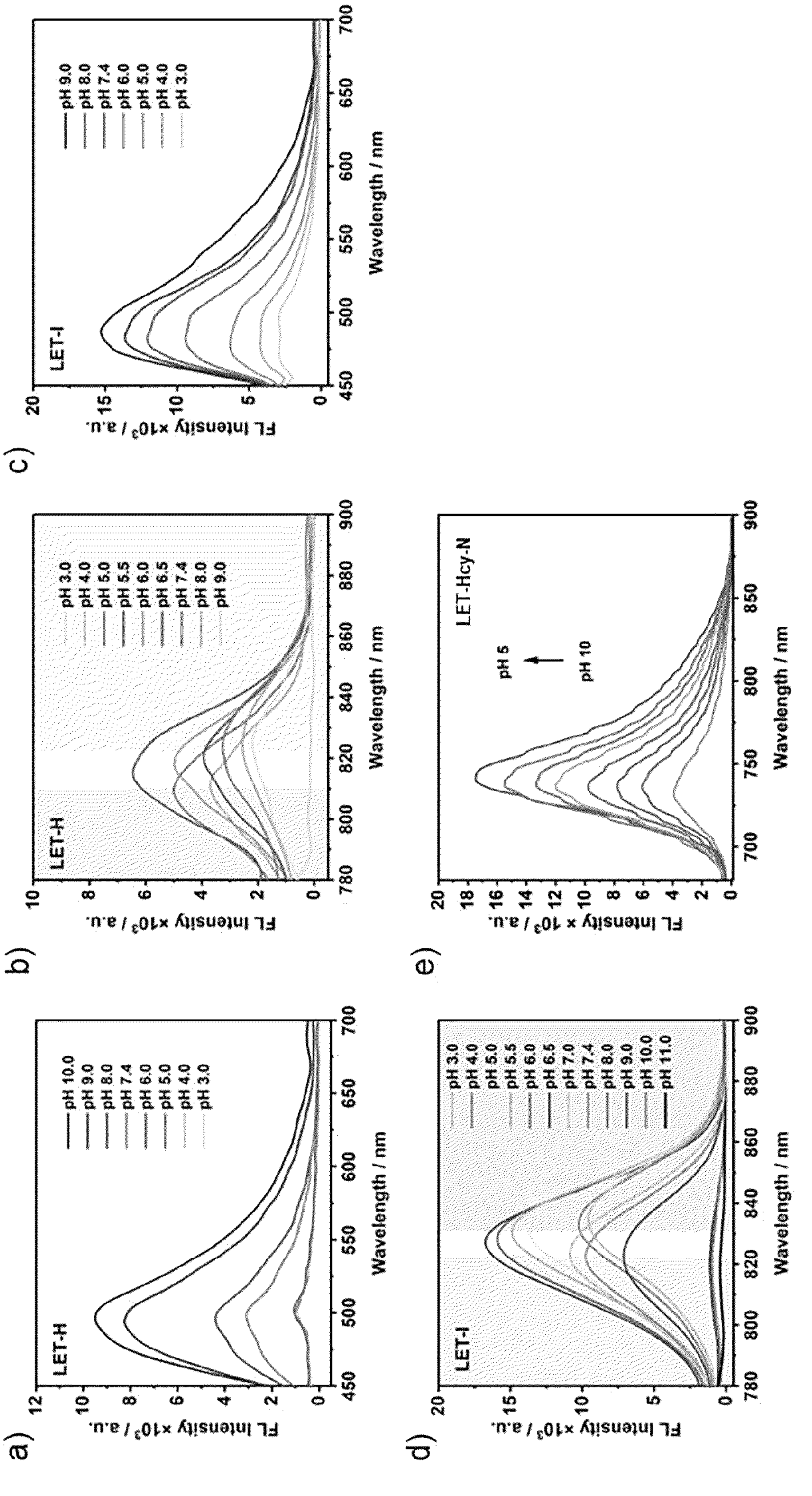
a)
LET-H
pH 10.0
pH 9.0
pH 8.0
pH 7.4
pH 6.0
pH 5.0
pH 4.0
pH 3.0
FL Intensity ×10$^3$ / a.u.
Wavelength / nm
b)
LET-H
pH 3.0
pH 4.0
pH 5.0
pH 5.5
pH 6.0
pH 6.5
pH 7.4
pH 8.0
pH 9.0
FL Intensity ×10$^3$ / a.u.
Wavelength / nm
c)
LET-I
pH 9.0
pH 8.0
pH 7.4
pH 6.0
pH 5.0
pH 4.0
pH 3.0
FL Intensity ×10$^3$ / a.u.
Wavelength / nm
d)
LET-I
pH 3.0
pH 4.0
pH 5.0
pH 5.5
pH 6.0
pH 6.5
pH 7.0
pH 7.4
pH 8.0
pH 9.0
pH 10.0
pH 11.0
FL Intensity ×10$^3$ / a.u.
Wavelength / nm
e)
LET-Hcy-N
pH 5
pH 10
FL Intensity × 10$^3$ / a.u.
Wavelength / nm

FIG. 7

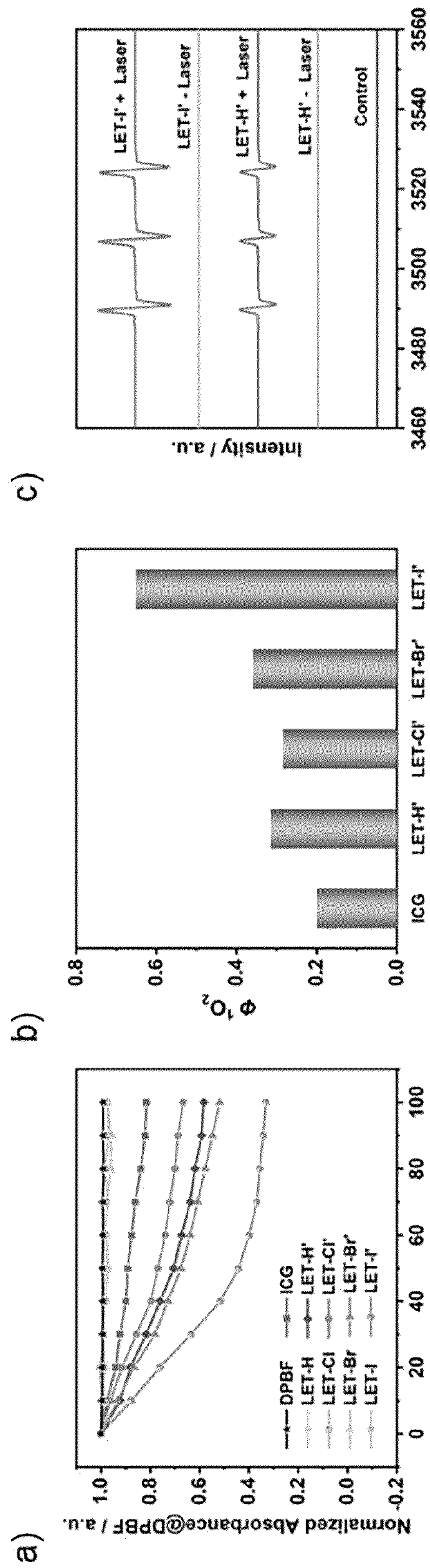
a)
Normalized Absorbance@DPBF / a.u.
Time / s
DPBF
LET-H
LET-Cl
LET-Br
LET-I
ICG
LET-H'
LET-Cl'
LET-Br'
LET-I'
b)
Φ $^1O_2$
ICG
LET-H'
LET-Cl'
LET-Br'
LET-I'
c)
Intensity / a.u.
Magnetic Field / G
LET-I' + Laser
LET-I' - Laser
LET-H' + Laser
LET-H' - Laser
Control

FIG. 8

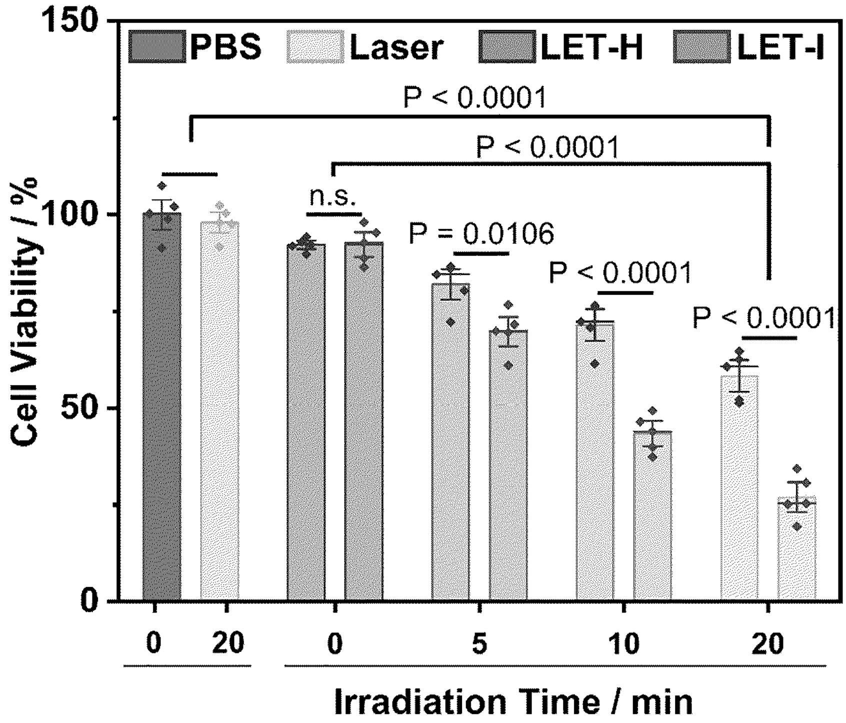
PBS
Laser
LET-H
LET-I
P < 0.0001
P < 0.0001
n.s.
P = 0.0106
P < 0.0001
P < 0.0001
Cell Viability / %
150
100
50
0
0
20
0
5
10
20
Irradiation Time / min

FIG. 9

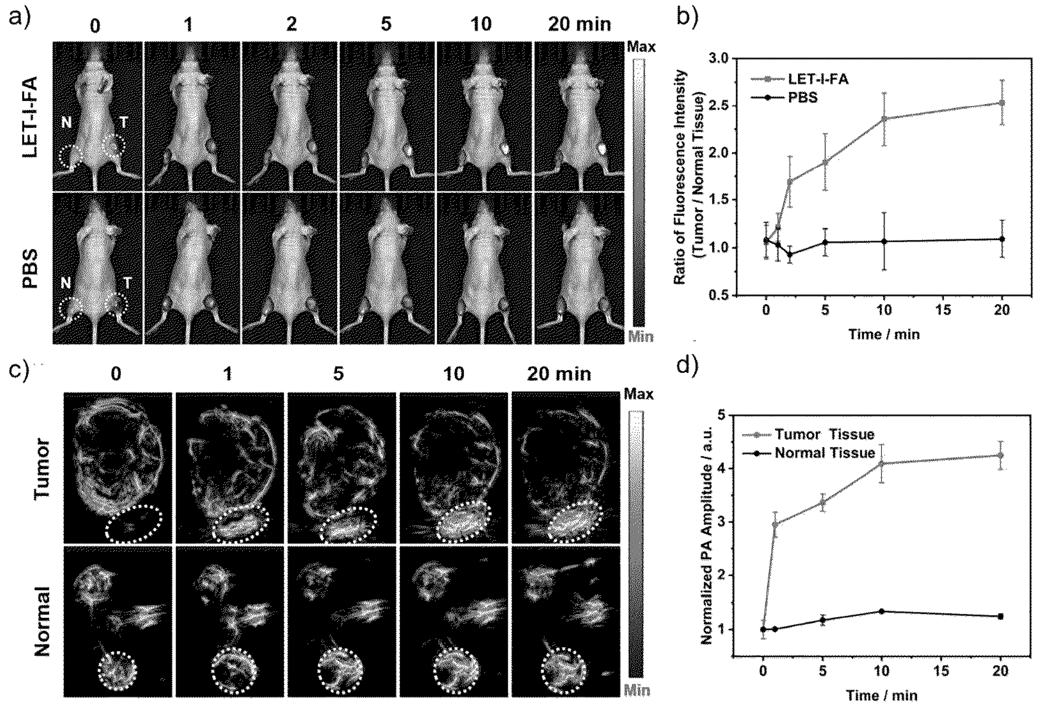
a)
0
1
2
5
10
20 min
LET-I-FA
PBS
N
T
Max
Min
b)
LET-I-FA
PBS
Ratio of Fluorescence Intensity (Tumor / Normal Tissue)
Time / min
c)
0
1
5
10
20 min
Tumor
Normal
Max
Min
d)
Tumor Tissue
Normal Tissue
Normalized PA Amplitude / a.u.
Time / min

FIG. 10

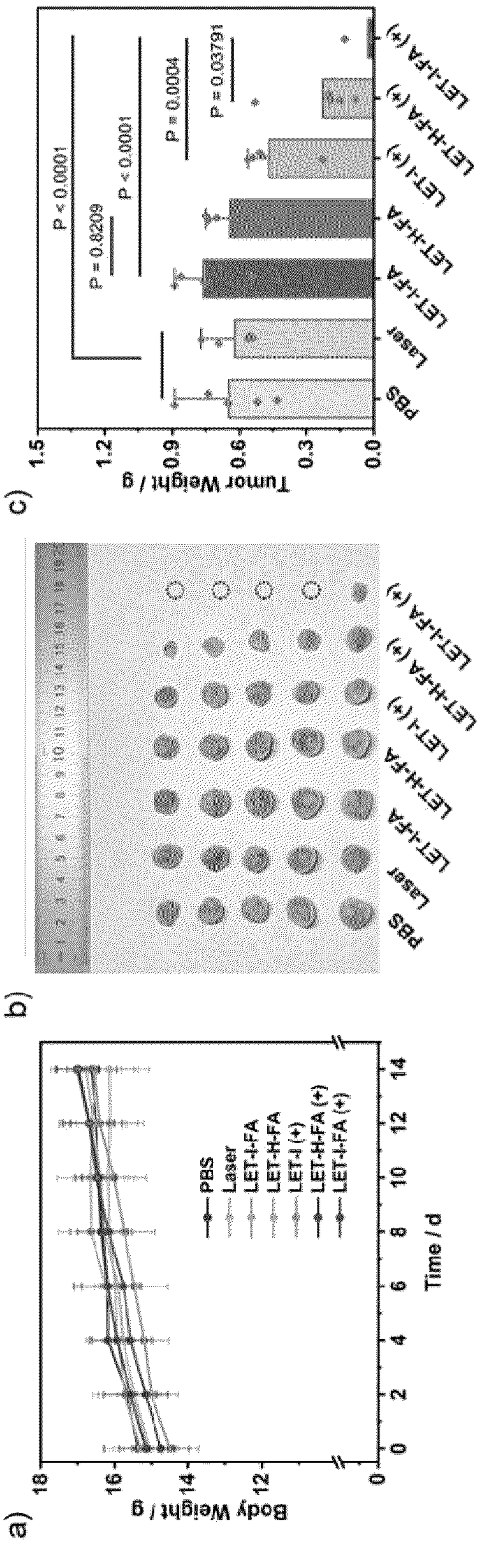
a)
Body Weight / g
Time / d
PBS
Laser
LET-I-FA
LET-H-FA
LET-I (+)
LET-H-FA (+)
LET-I-FA (+)
b)
PBS
Laser
LET-I-FA
LET-H-FA
LET-I (+)
LET-H-FA (+)
LET-I-FA (+)
c)
Tumor Weight / g
P < 0.0001
P = 0.8209
P < 0.0001
P = 0.0004
P = 0.03791
PBS
Laser
LET-I-FA
LET-H-FA
LET-I (+)
LET-H-FA (+)
LET-I-FA (+)

FIG. 11

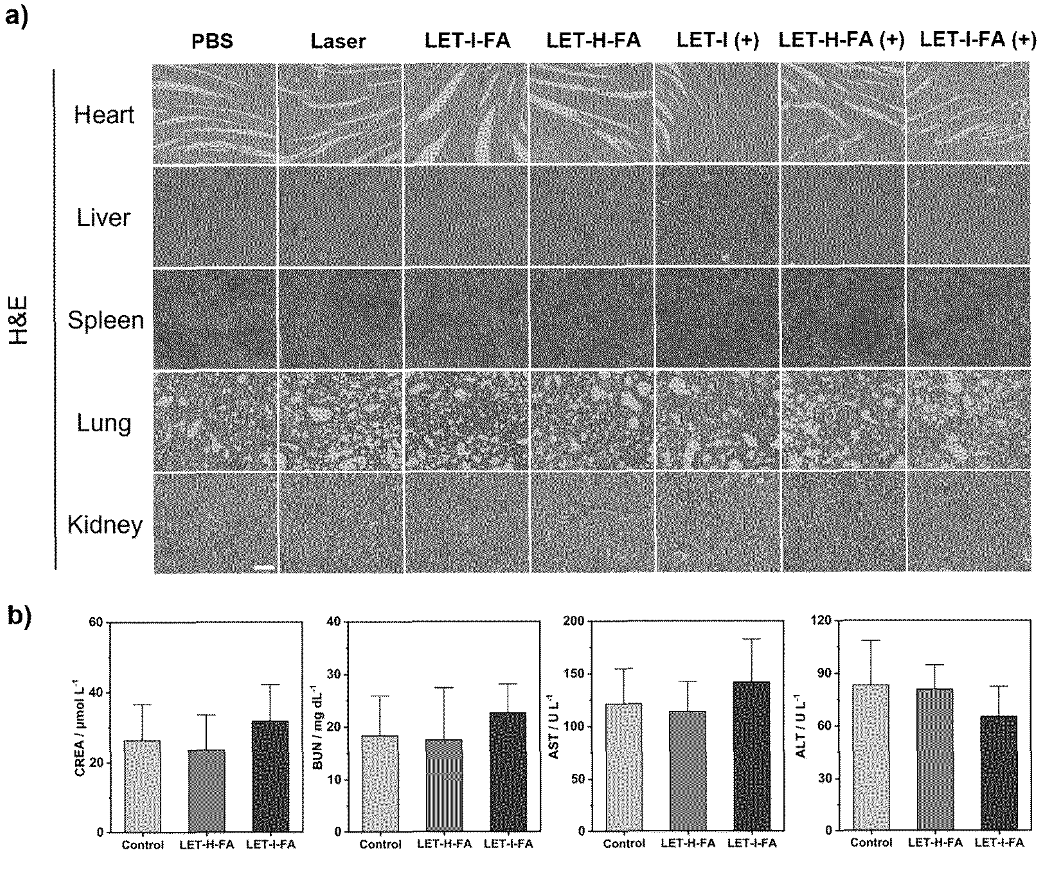
a)
PBS
Laser
LET-I-FA
LET-H-FA
LET-I (+)
LET-H-FA (+)
LET-I-FA (+)
H&E
Heart
Liver
Spleen
Lung
Kidney
b)
CREA / μmol L⁻¹
BUN / mg dL⁻¹
AST / U L⁻¹
ALT / U L⁻¹
Control
LET-H-FA
LET-I-FA

FIG. 12

**INTERNATIONAL SEARCH REPORT**

International application No.
**PCT/CN2025/091672**

**A. CLASSIFICATION OF SUBJECT MATTER**

C07D 498/04(2006.01)i; C07D 498/06(2006.01)i; A61K 41/00(2020.01)i; A61P 35/00(2006.01)i; A61K 49/22(2006.01)i; A61K 49/00(2006.01)i; G01N 21/64(2006.01)i

According to International Patent Classification (IPC) or to both national classification and IPC

**B. FIELDS SEARCHED**

Minimum documentation searched (classification system followed by classification symbols)

IPC: C07D 498/-,A61K,A61P

Documentation searched other than minimum documentation to the extent that such documents are included in the fields searched

Electronic data base consulted during the international search (name of data base and, where practicable, search terms used)

CNKI, CNABS, CNTXT, ENTXT, WPABS, REGISTRY, CAPLUS, MARPAT: 近红外, 光敏剂, 肿瘤, tumor, near, infrared, switch, 根据权利要求1进行的结构式检索, structural formula search conducted according to claim 1.

**C. DOCUMENTS CONSIDERED TO BE RELEVANT**

| Category* | Citation of document, with indication, where appropriate, of the relevant passages | Relevant to claim No. |
|---|---|---|
| PX | CN 118580253 A (SHENZHEN UNIVERSITY) 03 September 2024 (2024-09-03)<br>entire document, in particular, claims 1-10 | 1-10 |
| PA | CN 119874721 A (SHENZHEN UNIVERSITY) 25 April 2025 (2025-04-25)<br>entire document, in particular, claims 1-10 | 1-10 |
| A | QI, Qingkai et al. "Force-Induced Near-Infrared Chromism of Mechanophore-Linked Polymers"<br>*Journal of the American Chemical Society,*<br>Vol. 143, No. 42, 29 September 2021 (2021-09-29), 17337-17343<br>scheme1 | 1-10 |
| A | CN 113493465 A (GUANGDONG UNIVERSITY OF PETROCHEMICAL TECHNOLOGY) 12 October 2021 (2021-10-12)<br>entire document | 1-10 |
| A | CHEN, Peng et al. "Design, Synthesis and Properties of Near-infrared Molecular Switches Containing a Fluorene Ring"<br>*Organic & Biomolecular Chemistry,* Vol. 14, No. 19, 05 April 2016 (2016-04-05), 4456-4463<br>entire document | 1-10 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 June 2025** | **24 June 2025** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2025/091672**

| Patent document cited in search report | Publication date (day/month/year) | Patent family member(s) | Publication date (day/month/year) |
|---|---|---|---|
| CN 118580253 A | 03 September 2024 | None | |
| CN 119874721 A | 25 April 2025 | None | |
| CN 113493465 A | 12 October 2021 | CN 113493465 B | 02 September 2022 |

Form PCT/ISA/210 (patent family annex) (July 2022)